# EUROPEAN PATENT APPLICATION

(11) **EP 1 967 588 A2**
(43) Date of publication of application: **10.09.2008**
(21) Application number: 07023212.9
(22) Date of filing: 17.12.1999
(51) Int. Cl.: C12N 15/82, A01H 5/00

(54) **S-adenosyl-L-methionine synthetase promoter and its use in expression of transgenic genes in plants**

(30) Priority: 21.12.1998 US 113045 P
(62) Divisional of application: 99968137.2
(71) Applicant: E.I. DU PONT DE NEMOURS AND COMPANY, Wilmington DE 19898 (US)
(72) Inventor: Falco, Saverio Carl, Arden, DE 19810 (US); Li, Zhongsen, Hockessin, DE 19707 (US)
(74) Representative: Webber, Philip Michael

(57) **Abstract**

A constitutive plant S-adenosyl-L-methionine synthetase (SAMS) promoter and subfragments thereof and their use in promoting the expression of one or more heterologous nucleic acid fragments in plants are described.

SEQUENCE LISTING

<110> E. I. du Pont de Nemours and Company

<120> S-ADENOSYL-L-METHIONINE SYNTHETASE PROMOTER AND
ITS USE IN EXPRESSION OF TRANSGENIC GENES IN PLANTS

<130> BB1205

<140>
<141>

<150> 60/113,045
<151> 1998-12-21

<160> 16

<170> Microsoft Office 97

<210> 1
<211> 1518
<212> DNA
<213> Glycine max

<400> 1
agccaagccc cactcaacca ccacaccact ctctctgctc ttcttctacc tttcaagttt 60
ttaaagtatt aagatggcag agacattcct atttacctca gagtcagtga acgagggaca 120
ccctgacaag ctctgcgacc aaatctccga tgctgtcctc gacgcttgcc ttgaacagga 180
cccagacagc aaggttgcct gcgaaacatg caccaagacc aacttggtca tggtcttcgg 240
agagatcacc accaaggcca acgttgacta cgagaagatc gtgcgtgaca cctgcaggaa 300
catcggcttc gtctcaaacg atgtgggact tgatgctgac aactgcaagg tccttgtaaa 360
cattgagcag cagagccctg atattgccca gggtgtgcac ggccacctta ccaaaagacc 420
cgaggaaatc ggtgctggag accagggtca catgtttggc tatgccacgg acgaaacccc 480
agaattgatg ccattgagtc atgttcttgc aactaaactc ggtgctcgtc tcaccgaggt 540
tcgcaagaac ggaacctgcc catggttgag gcctgatggg aaaacccaag tgactgttga 600
gtattacaat gacaacggtg ccatggttcc agttcgtgtc cacactgtgc ttatctccac 660
ccaacatgat gagactgtga ccaacgacga aattgcagct gacctcaagg agcatgtgat 720
caagccggtg atcccggaga agtaccttga tgagaagacc attttccact tgaacccctc 780
tggccgtttt gtcattggag gtcctcacgg tgatgctggt ctcaccggcc gcaagatcat 840
catcgatact tacggaggat ggggtgctca tggtggtggt gctttctccg ggaaggatcc 900
caccaaggtt gataggagtg gtgcttacat tgtgagacag gctgctaaga gcattgtggc 960
aagtggacta gccagaaggt gcattgtgca agtgtcttat gccattggtg tgcccgagcc 1020
tttgtctgtc tttgttgaca cctatggcac cgggaagatc catgataagg agattctcaa 1080
cattgtgaag gagaactttg atttcaggcc cggtatgatc tccatcaacc ttgatctcaa 1140
gaggggtggg aataacaggt tcttgaagac tgctgcatat ggacacttcg gcagagagga 1200
ccctgacttc acatgggaag tggtcaagcc cctcaagtgg gagaaggcct aaggccattc 1260
attccactgc aatgtgctgg gagtttttta gcgttgccct tataatgtct attatccata 1320
actttccacg tcccttgctc tgtgtttttc tctcgtcgtc ctcctcctat tttgtttctc 1380
ctgcctttca tttgtaattt tttacatgat caactaaaaa atgtactctc tgttttccga 1440
ccattgtgtc tcttaatatc agtatcaaaa agaatgttcc aagttaaaaa aaaaaaaaaa 1500
aaaaaaaaaa aaaaaaaa 1518

<210> 2
<211> 2336
<212> DNA
<213> Glycine max

<400> 2
atcgatagag acatgttatt cacaaaccat aaaatgatgg ctaaaattgg tgtgattgga 60
acgatatctg tttattatga tttcagggcg caaaaatgcg agtacttaat aaaattttac 120
atttaaatta gaattttttt tatcaataaa tattaattta ttagttttat tagaaatatt 180
aattagaaaa ttttgaatcc ccgatttctc ctccttttct tcgctattca tcattttcta 240
accaaaccaa tcttatatgt tcttcaaatt agaacttgaa attattaatt ataattaaac 300
tgaaaacaat ttggtatcaa ttcatataca tgcttagtaa taaaatgcga taattaattg 360
ataaatctgc aaaagatttt acaaatatct ttcagaaaaa attaataaca aattttgtcg 420
ttttcatggt gttggtctga ggaggatttg gcactataga actctcctac ggaccattct 480
ttgcacttca actaaacgat ggtcagaatt ggtggggatt ttatattcaa gcatatccct 540
ttcaaaactt cctacttact tcgtgcgttc ggtaatcggt aacattagac tttcaaaatc 600
atttttaacc cctaaacagt aaatttgaag gacaaaaata atatttttca aatttgatag 660
actatttttt ttttgtaatt tgacgaacca aaaccagatt tatcctgaat tttaggaacc 720
acagatgtaa ctaaaccaat atttatttat tttctaaaac aaaatttcat ggcagcatgc 780
ctcagcccat gaaaaaaacc ttataaaaat atctacacat tgaccattga aaagttcgtt 840
ctcccatggg taaccagatc aaactcacat ccaaacataa catggatatc tccttaccaa 900
tcatactaat tattttgggt taaatattaa tcattatttt taagatatta attaagaaat 960
taaaagattt tttaaaaaaa tgtataaaat tatattattc atgatttttc atacatttga 1020
ttttgataat aaatatattt tttttaattt cttaaaaaat gttgcaagac acttattaga 1080
catagtcttg ttctgtttac aaaagcattc atcatttaat acattaaaaa atatttaata 1140
ctaacagtag aatcttcttg tgagtggtgt gggagtaggc aacctggcat tgaaacgaga 1200
gaaagagagt cagaaccaga agacaaataa aaagtatgca acaaacaaat caaaatcaaa 1260
gggcaaaggc tggggttggc tcaattggtt gctacattca attttcaact cagtcaacgg 1320
ttgagattca ctctgacttc cccaatctaa gccgcggatg caaacggttg aatctaaccc 1380
acaatccaat ctcgttactt aggggctttt ccgtcattaa ctcacccctg ccacccggtt 1440
tccctataaa ttggaactca atgctcccct ctaaactcgt atcgcttcag agttgagacc 1500
aagacacact cgttcatata tctctctgct cttctcttct cttctacctc tcaaggtact 1560
tttcttctcc ctctaccaaa tcctagattc cgtggttcaa tttcggatct tgcacttctg 1620
gtttgctttg ccttgctttt tcctcaactg ggtccatcta ggatccatgt gaaactctac 1680
tctttcttta atatctgcgg aatacgcgtt ggactttcag atctagtcga aatcatttca 1740
taattgcctt tctttctttt agcttatgag aaataaaatc attttttttt atttcaaaat 1800
aaaccttggg ccttgtgctg actgagatgg ggtttggtga ttacagaatt ttagcgaatt 1860
ttgtaattgt acttgtttgt ctgtagtttt gttttgtttt cttgtttctc atacattcct 1920
taggcttcaa ttttattcga gtataggtca caataggaat tcaaactttg agcaggggaa 1980
ttaatccctt ccttcaaatc cagtttgttt gtatatatgt ttaaaaaatg aaacttttgc 2040
tttaaattct attataactt tttttatggc aaaaattttt gcatgtgtct ttgctctcct 2100
gttgtaaatt tactgtttag gtactaactc taggcttgtt gtgcagtttt tgaagtataa 2160
agatggcaga gacattccta ttcacctcgg agtcagtgaa cgagggacac cctgataagc 2220
tctgcgacca aatctccgat gctgtcctcg acgcttgcct cgaacaggac ccagacagca 2280
aggttgcctg cgaaacatgc accaagacca acttggtcat ggtcttcgga gagatc 2336

<210> 3
<211> 522
<212> DNA
<213> Glycine max

<220>
<221> unsure
<222> (405)

<220>
<221> unsure
<222> (509)

<220>
<221> unsure
<222> (515)

<400> 3
gaccaagaca cactcgttca tatatctctc tgctcttctc ttctcttcta cctctcaagt 60
ttttgaagta taaagatggc agagacattc ctattcacct cggagtcagt gaacgaggga 120
caccctgata agctctgcga ccaaatctcc gatgctgtcc tcgacgcttg cctcgaacag 180
gacccagaca gcaaggttgc ctgcgaaaca tgcaccaaga ccaacttggt catggtcttc 240
ggagagatca ccaccaaggc caacgttgac tacgagaaga tcgtgcgtga cacctgcagg 300
agcatcggct tcatctcaaa cgatgtggga cttgatgctg acaactgcaa ggtccttgta 360
aacattgagc agcagagccc tgatattgcc cagggcgtgc acggncacct taccaaaaga 420
cctgaagaaa ttggcgctgg tgaccaaggt cacatgtttg gctatgccac tgatgaaacc 480
ccaaaattca tgccattgag tcatgttcnt gcaancaagc tc 522

<210> 4
<211> 32
<212> DNA
<213> Artificial Sequence

<220>
<223> Description of Artificial Sequence: PCR Primer

<400> 4
catgccatgg ctttatactt caaaaactgc ac 32

<210> 5
<211> 24
<212> DNA
<213> Artificial Sequence

<220>
<223> Description of Artificial Sequence: PCR Primer

<400> 5
gctctagatc aaactcacat ccaa 24

<210> 6
<211> 1314
<212> DNA
<213> Glycine max

<400> 6
tctagatcaa actcacatcc aaacataaca tggatatctc cttaccaatc atactaatta 60
ttttgggtta aatattaatc attattttta agatattaat taagaaatta aaagattttt 120
taaaaaaatg tataaaatta tattattcat gatttttcat acatttgatt ttgataataa 180
atatattttt tttaatttct taaaaaatgt tgcaagacac ttattagaca tagtcttgtt 240
ctgtttacaa aagcattcat catttaatac attaaaaaat atttaatact aacagtagaa 300
tcttcttgtg agtggtgtgg gagtaggcaa cctggcattg aaacgagaga aagagagtca 360
gaaccagaag acaaataaaa agtatgcaac aaacaaatca aaatcaaagg gcaaaggctg 420
gggttggctc aattggttgc tacattcaat tttcaactca gtcaacggtt gagattcact 480
ctgacttccc caatctaagc cgcggatgca aacggttgaa tctaacccac aatccaatct 540
cgttacttag gggcttttcc gtcattaact cacccctgcc acccggtttc cctataaatt 600
ggaactcaat gctcccctct aaactcgtat cgcttcagag ttgagaccaa gacacactcg 660
ttcatatatc tctctgctct tctcttctct tctacctctc aaggtacttt tcttctccct 720
ctaccaaatc ctagattccg tggttcaatt tcggatcttg cacttctggt ttgctttgcc 780
ttgctttttc ctcaactggg tccatctagg atccatgtga aactctactc tttctttaat 840
atctgcggaa tacgcgttgg actttcagat ctagtcgaaa tcatttcata attgcctttc 900
tttcttttag cttatgagaa ataaaatcat ttttttttat ttcaaaataa accttgggcc 960
ttgtgctgac tgagatgggg tttggtgatt acagaatttt agcgaatttt gtaattgtac 1020
ttgtttgtct gtagttttgt tttgttttct tgtttctcat acattcctta ggcttcaatt 1080
ttattcgagt ataggtcaca ataggaattc aaactttgag caggggaatt aatcccttcc 1140
ttcaaatcca gtttgtttgt atatatgttt aaaaaatgaa acttttgctt taaattctat 1200

tataactttt tttatggcaa aaatttttgc atgtgtcttt gctctcctgt tgtaaattta 1260
ctgtttaggt actaactcta ggcttgttgt gcagtttttg aagtataacc atgg 1314

<210> 7
<211> 22
<212> DNA
<213> Artificial Sequence

<220>
<223> Description of Artificial Sequence: PCR Primer

<400> 7
ttcgagtata ggtcacaata gg 22

<210> 8
<211> 19
<212> DNA
<213> Artificial Sequence

<220>
<223> Description of Artificial Sequence: PCR Primer

<400> 8
cttcgctgag gacatggac 19

<210> 9
<211> 21
<212> DNA
<213> Artificial Sequence

<220>
<223> Description of Artificial Sequence: PCR Primer

<400> 9
gagttgtcgc tgttgttcga c 21

<210> 10
<211> 20
<212> DNA
<213> Artificial Sequence

<220>
<223> Description of Artificial Sequence: PCR Primer

<400> 10
aacacagcat ccgcattgcg 20

<210> 11
<211> 21
<212> DNA
<213> Artificial Sequence

<220>
<223> Description of Artificial Sequence: PCR Primer

<400> 11
aggagtgcag aatcagatca g 21

<210> 12
<211> 20

<212>  DNA
<213>  Artificial Sequence

<220>
<223>  Description of Artificial Sequence: PCR Primer

<400>  12
gctgatcgaa ccagatggag                                                20

<210>  13
<211>  23
<212>  DNA
<213>  Artificial Sequence

<220>
<223>  Description of Artificial Sequence: PCR Primer

<400>  13
ctgtacagtt aaacagtagt tct                                            23

<210>  14
<211>  2165
<212>  DNA
<213>  Glycine max

<400>  14
atcgatagag acatgttatt cacaaaccat aaaatgatgg ctaaaattgg tgtgattgga   60
acgatatctg tttattatga tttcagggcg caaaaatgcg agtacttaat aaaattttac  120
atttaaatta gaattttttt tatcaataaa tattaattta ttagttttat tagaaatatt  180
aattagaaaa ttttgaatcc ccgatttctc ctccttttct tcgctattca tcattttcta  240
accaaaccaa tcttatatgt tcttcaaatt agaacttgaa attattaatt ataattaaac  300
tgaaaacaat ttggtatcaa ttcatataca tgcttagtaa taaaatgcga taattaattg  360
ataaatctgc aaaagatttt acaaatatct ttcagaaaaa attaataaca aattttgtcg  420
ttttcatggt gttggtctga ggaggatttg gcactataga actctcctac ggaccattct  480
ttgcacttca actaaacgat ggtcagaatt ggtggggatt ttatattcaa gcatatccct  540
ttcaaaactt cctacttact tcgtgcgttc ggtaatcggt aacattagac tttcaaaatc  600
atttttaacc cctaaacagt aaatttgaag gacaaaaata atatttttca aatttgatag  660
actatttttt ttttgtaatt tgacgaacca aaaccagatt tatcctgaat tttaggaacc  720
acagatgtaa ctaaaccaat atttatttat tttctaaaac aaaatttcat ggcagcatgc  780
ctcagcccat gaaaaaaacc ttataaaaat atctacacat tgaccattga aaagttcgtt  840
ctcccatggg taaccagatc aaactcacat ccaaacataa catggatatc tccttaccaa  900
tcatactaat tattttgggt taaatattaa tcattatttt taagatatta attaagaaat  960
taaaagattt tttaaaaaaa tgtataaaat tatattattc atgatttttc atacatttga 1020
ttttgataat aaatatattt tttttaattt cttaaaaaat gttgcaagac acttattaga 1080
catagtcttg ttctgtttac aaaagcattc atcatttaat acattaaaaa atatttaata 1140
ctaacagtag aatcttcttg tgagtggtgt gggagtaggc aacctggcat tgaaacgaga 1200
gaaagagagt cagaaccaga agacaaataa aaagtatgca acaaacaaat caaaatcaaa 1260
gggcaaaggc tggggttggc tcaattggtt gctacattca attttcaact cagtcaacgg 1320
ttgagattca ctctgacttc cccaatctaa gccgcggatg caaacggttg aatctaaccc 1380
acaatccaat ctcgttactt aggggctttt ccgtcattaa ctcacccctg ccacccggtt 1440
tccctataaa ttggaactca atgctcccct ctaaactcgt atcgcttcag agttgagacc 1500
aagacacact cgttcatata tctctctgct cttctcttct cttctacctc tcaaggtact 1560
tttcttctcc ctctaccaaa tcctagattc cgtggttcaa tttcggatct tgcacttctg 1620
gtttgctttg ccttgctttt tcctcaactg ggtccatcta ggatccatgt gaaactctac 1680
tctttcttta atatctgcgg aatacgcgtt ggactttcag atctagtcga aatcatttca 1740
taattgcctt tctttctttt agcttatgag aaataaaatc attttttttt atttcaaaat 1800
aaaccttggg ccttgtgctg actgagatgg ggtttggtga ttacagaatt ttagcgaatt 1860
ttgtaattgt acttgtttgt ctgtagtttt gttttgtttt cttgtttctc atacattcct 1920
taggcttcaa ttttattcga gtataggtca caataggaat tcaaactttg agcaggggaa 1980
ttaatccctt ccttcaaatc cagtttgttt gtatatatgt ttaaaaaatg aaacttttgc 2040

tttaaattct attataactt tttttatggc aaaaattttt gcatgtgtct ttgctctcct 2100
gttgtaaatt tactgtttag gtactaactc taggcttgtt gtgcagtttt tgaagtataa 2160
agatg 2165

<210> 15
<211> 1574
<212> DNA
<213> Glycine max

<400> 15
atcgatagag acatgttatt cacaaaccat aaaatgatgg ctaaaattgg tgtgattgga 60
acgatatctg tttattatga tttcagggcg caaaaatgcg agtacttaat aaaattttac 120
atttaaatta gaattttttt tatcaataaa tattaattta ttagttttat tagaaatatt 180
aattagaaaa ttttgaatcc ccgatttctc ctccttttct tcgctattca tcattttcta 240
accaaaccaa tcttatatgt tcttcaaatt agaacttgaa attattaatt ataattaaac 300
tgaaaacaat ttggtatcaa ttcatataca tgcttagtaa taaaatgcga taattaattg 360
ataaatctgc aaaagatttt acaaatatct ttcagaaaaa attaataaca aattttgtcg 420
ttttcatggt gttggtctga ggaggatttg gcactataga actctcctac ggaccattct 480
ttgcacttca actaaacgat ggtcagaatt ggtggggatt ttatattcaa gcatatccct 540
ttcaaaactt cctacttact tcgtgcgttc ggtaatcggt aacattagac tttcaaaatc 600
atttttaacc cctaaacagt aaatttgaag gacaaaaata atatttttca aatttgatag 660
actatttttt ttttgtaatt tgacgaacca aaaccagatt tatcctgaat tttaggaacc 720
acagatgtaa ctaaaccaat atttatttat tttctaaaac aaaatttcat ggcagcatgc 780
ctcagcccat gaaaaaaacc ttataaaaat atctacacat tgaccattga aaagttcgtt 840
ctcccatggg taaccagatc aaactcacat ccaaacataa catggatatc tccttaccaa 900
tcatactaat tattttgggt taaatattaa tcattatttt taagatatta attaagaaat 960
taaaagattt tttaaaaaaa tgtataaaat tatattattc atgatttttc atacatttga 1020
ttttgataat aaatatattt tttttaattt cttaaaaaat gttgcaagac acttattaga 1080
catagtcttg ttctgtttac aaaagcattc atcatttaat acattaaaaa atatttaata 1140
ctaacagtag aatcttcttg tgagtggtgt gggagtaggc aacctggcat tgaaacgaga 1200
gaaagagagt cagaaccaga agacaaataa aaagtatgca acaaacaaat caaaatcaaa 1260
gggcaaaggc tggggttggc tcaattggtt gctacattca attttcaact cagtcaacgg 1320
ttgagattca ctctgacttc cccaatctaa gccgcggatg caaacggttg aatctaaccc 1380
acaatccaat ctcgttactt aggggctttt ccgtcattaa ctcacccctg ccacccggtt 1440
tccctataaa ttggaactca atgctcccct ctaaactcgt atcgcttcag agttgagacc 1500
aagacacact cgttcatata tctctctgct cttctcttct cttctacctc tcaagttttt 1560
gaagtataaa gatg 1574

<210> 16
<211> 719
<212> DNA
<213> Glycine max

<400> 16
agatcaaact cacatccaaa cataacatgg atatctcctt accaatcata ctaattattt 60
tgggttaaat attaatcatt atttttaaga tattaattaa gaaattaaaa gattttttaa 120
aaaaatgtat aaaattatat tattcatgat tttcatacat tttgattttg ataataaata 180
tatttttttt aatttcttaa aaaatgttgc aagacactta ttagacatag tcttgttctg 240
tttacaaaag cattcatcat ttaatacatt aaaaaatatt taatactaac agtagaatct 300
tcttgtgagt ggtgtgggag taggcaacct ggcattgaaa cgagagaaag agagtcagaa 360
ccagaagaca aataaaaagt atgcaacaaa caaatcaaaa tcaaagggca aaggctgggg 420
ttggctcaat tggttgctac attcaatttt caactcagtc aacggttgag attcactctg 480
acttccccaa tctaagccgc ggatgcaaac ggttgaatct aacccacaat ccaatctcgt 540
tacttagggg cttttccgtc attaactcac ccctgccacc cggtttccct ataaattgga 600
actcaatgct cccctctaaa ctcgtatcgc ttcagagttg agaccaagac acactcgttc 660
atatatctct ctgctcttct cttctcttct acctctcaag tttttgaagt ataaagatg 719

<210> 17
<211> 6975

<212>  DNA
<213>  Artificial Sequence

<220>
<223>  Description of Artificial Sequence:plasmid

<400>  17
gaatatgcat cactagtaag ctttgctcta gaggatccaa ttccaatccc acaaaaatct   60
gagcttaaca gcacagttgc tcctctcaga gcagaatcgg gtattcaaca ccctcatatc  120
aactactacg ttgtgtataa cggtccacat gccggtatat acgatgactg gggttgtaca  180
aaggcggcaa caaacggcgt tcccggagtt gcacacaaga aatttgccac tattacagag  240
gcaagagcag cagctgacgc gtacacaaca agtcagcaaa cagacaggtt gaacttcatc  300
cccaaaggag aagctcaact caagcccaag agctttgcta aggccctaac aagcccacca  360
aagcaaaaag cccactggct cacgctagga accaaaaggc ccagcagtga tccagcccca  420
aaagagatct cctttgcccc ggagattaca atggacgatt tcctctatct ttacgatcta  480
ggaaggaagt tcgaaggtga aggtgacgac actatgttca ccactgataa tgagaaggtt  540
agcctcttca atttcagaaa gaatgctgac ccacagatgg ttagagaggc ctacgcagca  600
ggtctcatca agacgatcta cccgagtaac aatctccagg agatcaaata ccttcccaag  660
aaggttaaag atgcagtcaa aagattcagg actaattgca tcaagaacac agagaaagac  720
atatttctca agatcagaag tactattcca gtatggacga ttcaaggctt gcttcataaa  780
ccaaggcaag taatagagat tggagtctct aaaaaggtag ttcctactga atctaaggcc  840
atgcatggag tctaagattc aaatcgagga tctaacagaa ctcgccgtga agactggcga  900
acagttcata cagagtcttt tacgactcaa tgacaagaag aaaatcttcg tcaacatggt  960
ggagcacgac actctggtct actccaaaaa tgtcaaagat acagtctcag aagaccaaag 1020
ggctattgag acttttcaac aaaggataat ttcgggaaac ctcctcggat tccattgccc 1080
agctatctgt cacttcatcg aaaggacagt agaaaaggaa ggtggctcct acaaatgcca 1140
tcattgcgat aaaggaaagg ctatcattca agatgcctct gccgacagtg gtcccaaaga 1200
tggaccccca cccacgagga gcatcgtgga aaaagaagac gttccaacca cgtcttcaaa 1260
gcaagtggat tgatgtgaca tctccactga cgtaagggat gacgcacaat cccactatcc 1320
ttcgcaagac ccttcctcta tataaggaag ttcatttcat ttggagagga cacgctcgag 1380
ctcatttctc tattacttca gccataacaa aagaactctt ttctcttctt attaaaccat 1440
ggtacgtcct gtagaaaccc caacccgtga aatcaaaaaa ctcgacggcc tgtgggcatt 1500
cagtctggat cgcgaaaact gtggaattga tcagcgttgg tgggaaagcg cgttacaaga 1560
aagccgggca attgctgtgc caggcagttt taacgatcag ttcgccgatg cagatattcg 1620
taattatgcg ggcaacgtct ggtatcagcg cgaagtcttt ataccgaaag gttgggcagg 1680
ccagcgtatc gtgctgcgtt tcgatgcggt cactcattac ggcaaagtgt gggtcaataa 1740
tcaggaagtg atggagcatc agggcggcta tacgccattt gaagccgatg tcacgccgta 1800
tgttattgcc gggaaaagtg tacgtatcac cgtttgtgtg aacaacgaac tgaactggca 1860
gactatcccg ccgggaatgg tgattaccga cgaaaacggc aagaaaaagc agtcttactt 1920
ccatgatttc tttaactatg ccggaatcca tcgcagcgta atgctctaca ccacgccgaa 1980
cacctgggtg gacgatatca ccgtggtgac gcatgtcgcg caagactgta accacgcgtc 2040
tgttgactgg caggtggtgg ccaatggtga tgtcagcgtt gaactgcgtg atgcggatca 2100
acaggtggtt gcaactggac aaggcactag cgggactttg caagtggtga atccgcacct 2160
ctggcaaccg ggtgaaggtt atctctatga actgtgcgtc acagccaaaa gccagacaga 2220
gtgtgatatc tacccgcttc gcgtcggcat ccggtcagtg gcagtgaagg gccaacagtt 2280
cctgattaac cacaaaccgt tctactttac tggctttggt cgtcatgaag atgcggactt 2340
acgtggcaaa ggattcgata acgtgctgat ggtgcacgac cacgcattaa tggactggat 2400
tggggccaac tcctaccgta cctcgcatta cccttacgct gaagagatgc tcgactgggc 2460
agatgaacat ggcatcgtgg tgattgatga aactgctgct gtcggcttta acctctcttt 2520
aggcattggt ttcgaagcgg gcaacaagcc gaaagaactg tacagcgaag aggcagtcaa 2580
cggggaaact cagcaagcgc acttacaggc gattaaagag ctgatagcgc gtgacaaaaa 2640
ccacccaagc gtggtgatgt ggagtattgc caacgaaccg gatacccgtc cgcaagtgca 2700
cgggaatatt tcgccactgg cggaagcaac gcgtaaactc gacccgacgc gtccgatcac 2760
ctgcgtcaat gtaatgttct gcgacgctca caccgatacc atcagcgatc tctttgatgt 2820
gctgtgcctg aaccgttatt acggatggta tgtccaaagc ggcgatttgg aaacggcaga 2880
gaaggtactg gaaaaagaac ttctggcctg gcaggagaaa ctgcatcagc cgattatcat 2940
caccgaatac ggcgtggata cgttagccgg gctgcactca atgtacaccg acatgtggag 3000
tgaagagtat cagtgtgcat ggctggatat gtatcaccgc gtctttgatc gcgtcagcgc 3060
cgtcgtcggt gaacaggtat ggaatttcgc cgattttgcg acctcgcaag gcatattgcg 3120
cgttggcggt aacaagaaag ggatcttcac tcgcgaccgc aaaccgaagt cggcggcttt 3180

tctgctgcaa aaacgctgga ctggcatgaa cttcggtgaa aaaccgcagc agggaggcaa 3240
acaatgaatc aacaactctc ctggcgcacc atcgtcggct acagcctcgg tggggaattc 3300
cccgggggta cctaatagtg agatccaaca cttacgtttg caacgtccaa gagcaaatag 3360
accacgnacg ccggaaggtt gccgcagcgt gtggattgcg tctcaattct ctcttgcagg 3420
aatgcaatga tgaatatgat actgactatg aaactttgag ggaatactgc ctagcaccgt 3480
cacctcataa cgtgcatcat gcatgccctg acaacatgga acatcgctat ttttctgaag 3540
aattatgctc gttggaggat gtcgcggcaa ttgcagctat tgccaacatc gaactacccc 3600
tcacgcatgc attcatcaat attattcatg cggggaaagg caagattaat ccaactggca 3660
aatcatccag cgtgattggt aacttcagtt ccagcgactt gattcgtttt ggtgctaccc 3720
acgttttcaa taaggacgag atggtggagt aaagaaggag tgcgtcgaag cagatcgttc 3780
aaacatttgg caataaagtt tcttaagatt gaatcctgtt gccggtcttg cgatgattat 3840
catataattt ctgttgaatt acgttaagca tgtaataatt aacatgtaat gcatgacgtt 3900
atttatgaga tgggttttta tgattagagt cccgcaatta tacatttaat acgcgataga 3960
aaacaaaata tagcgcgcaa actaggataa attatcgcgc gcggtgtcat ctatgttact 4020
agatcgatca aacttcggta ctgtgtaatg acgatgagca atcgagaggc tgactaacaa 4080
aaggtacatc ggtcgacgag ctccctatag tgagtcgtat tagaggccga cttggccaaa 4140
ttcgtaatca tggtcatagc tgtttcctgt gtgaaattgt tatccgctca caattccaca 4200
caacatacga gccggaagca taaagtgtaa agcctggggt gcctaatgag tgagctaact 4260
cacattaatt gcgttgcgct cactgcccgc tttccagtcg ggaaacctgt cgtgccagct 4320
gcattaatga atcggccaac gcgcggggag aggcggtttg cgtattgggc gctcttccgc 4380
ttcctcgctc actgactcgc tgcgctcggt cgttcggctg cggcgagcgg tatcagctca 4440
ctcaaaggcg gtaatacggt tatccacaga atcaggggat aacgcaggaa agaacatgtg 4500
agcaaaaggc cagcaaaagg ccaggaaccg taaaaaggcc gcgttgctgg cgtttttcca 4560
taggctccgc ccccctgacg agcatcacaa aaatcgacgc tcaagtcaga ggtggcgaaa 4620
cccgacagga ctataaagat accaggcgtt tccccctgga agctccctcg tgcgctctcc 4680
tgttccgacc ctgccgctta ccggatacct gtccgccttt ctcccttcgg gaagcgtggc 4740
gctttctcat agctcacgct gtaggtatct cagttcggtg taggtcgttc gctccaagct 4800
gggctgtgtg cacgaacccc ccgttcagcc cgaccgctgc gccttatccg gtaactatcg 4860
tcttgagtcc aacccggtaa gacacgactt atcgccactg gcagcagcca ctggtaacag 4920
gattagcaga gcgaggtatg taggcggtgc tacagagttc ttgaagtggt ggcctaacta 4980
cggctacact agaaggacag tatttggtat ctgcgctctg ctgaagccag ttaccttcgg 5040
aaaaagagtt ggtagctctt gatccggcaa acaaaccacc gctggtagcg gtggtttttt 5100
tgtttgcaag cagcagatta cgcgcagaaa aaaaggatct caagaagatc ctttgatctt 5160
ttctacgggg tctgacgctc agtggaacga aaactcacgt taagggattt tggtcatgag 5220
attatcaaaa aggatcttca cctagatcct tttaaattaa aaatgaagtt ttaaatcaat 5280
ctaaagtata tatgagtaaa cttggtctga cagttaccaa tgcttaatca gtgaggcacc 5340
tatctcagcg atctgtctat ttcgttcatc catagttgcc tgactccccg tcgtgtagat 5400
aactacgata cgggagggct taccatctgg ccccagtgct gcaatgatac cgcgagaccc 5460
acgctcaccg gctccagatt tatcagcaat aaaccagcca gccggaaggg ccgagcgcag 5520
aagtggtcct gcaactttat ccgcctccat ccagtctatt aattgttgcc gggaagctag 5580
agtaagtagt tcgccagtta atagtttgcg caacgttgtt gccattgcta caggcatcgt 5640
ggtgtcacgc tcgtcgtttg gtatggcttc attcagctcc ggttcccaac gatcaaggcg 5700
agttacatga tcccccatgt tgtgcaaaaa agcggttagc tccttcggtc ctccgatcgt 5760
tgtcagaagt aagttggccg cagtgttatc actcatggtt atggcagcac tgcataattc 5820
tcttactgtc atgccatccg taagatgctt ttctgtgact ggtgagtact caaccaagtc 5880
attctgagaa tagtgtatgc ggcgaccgag ttgctcttgc ccggcgtcaa tacgggataa 5940
taccgcgcca catagcagaa ctttaaaagt gctcatcatt ggaaaacgtt cttcggggcg 6000
aaaactctca aggatcttac cgctgttgag atccagttcg atgtaaccca ctcgtgcacc 6060
caactgatct tcagcatctt ttactttcac cagcgtttct gggtgagcaa aaacaggaag 6120
gcaaaatgcc gcaaaaaagg gaataagggc gacacggaaa tgttgaatac tcatactctt 6180
cctttttcaa tattattgaa gcatttatca gggttattgt ctcatgagcg gatacatatt 6240
tgaatgtatt tagaaaaata aacaaatagg ggttccgcgc acatttcccc gaaaagtgcc 6300
acctgacgcg ccctgtagcg gcgcattaag cgcggcgggt gtggtggtta cgcgcagcgt 6360
gaccgctaca cttgccagcg ccctagcgcc cgctcctttc gctttcttcc cttcctttct 6420
cgccacgttc gccggctttc cccgtcaagc tctaaatcgg gcatcccttt agggttccga 6480
tttagtgctt tacggcacct cgaccccaaa aaacttgatt agggtgatgg ttcacgtagt 6540
gggccatcgc cctgatagac ggtttttcgc cctttgacgt tggagtccac gttctttaat 6600
agtggactct tgttccaaac tggaacaaca ctcaacccta tctcggtcta ttcttttgat 6660
ttataaggga ttttgccgat ttcggcctat tggttaaaaa atgagctgat ttaacaaaaa 6720
tttaacgcga attttaacaa aatattaacg aaatattaac gtttacaatt ttcccattcg 6780

ccattcaggc tgcgcaactg ttgggaaggg cgatcggtgc gggcctcttc gctattacgc 6840
cagctggcga aagggggatg tgctgcaagg cgattaagtt gggtaacgcc agggttttcc 6900
cagtcacgac gttgtaaaac gacggccagt gccaagctga cttggtcagc ggccgcagat 6960
ttaggtgaca ctata 6975

<210> 18
<211> 3985
<212> DNA
<213> Artificial Sequence

<220>
<223> Description of Artificial Sequence:chimeric gene

<400> 18
aagctttgct ctagatcaaa ctcacatcca aacataacat ggatatcttc cttaccaatc 60
atactaatta ttttgggtta aatattaatc attattttta agatattaat taagaaatta 120
aaagattttt taaaaaaatg tataaaatta tattattcat gatttttcat acatttgatt 180
ttgataataa atatattttt tttaatttct taaaaaatgt tgcaagacac ttattagaca 240
tagtcttgtt ctgtttacaa aagcattcat catttaatac attaaaaaat atttaatact 300
aacagtagaa tcttcttgtg agtggtgtgg gagtaggcaa cctggcattg aaacgagaga 360
aagagagtca gaaccagaag acaaataaaa agtatgcaac aaacaaatca aaatcaaagg 420
gcaaaggctg gggttggctc aattggttgc tacattcaat tttcaactca gtcaacggtt 480
gagattcact ctgacttccc caatctaagc cgcggatgca aacggttgaa tctaacccac 540
aatccaatct cgttacttag gggcttttcc gtcattaact cacccctgcc acccggtttc 600
cctataaatt ggaactcaat gctcccctct aaactcgtat cgcttcagag ttgagaccaa 660
gacacactcg ttcatatatc tctctgctct tctcttctct tctacctctc aaggtacttt 720
tcttctccct ctaccaaatc ctagattccg tggttcaatt tcggatcttg cacttctggt 780
ttgctttgcc ttgctttttc ctcaactggg tccatctagg atccatgtga aactctactc 840
tttctttaat atctgcggaa tacgcgttgg actttcagat ctagtcgaaa tcatttcata 900
attgcctttc tttcttttag cttatgagaa ataaaatcac ttttttttta tttcaaaata 960
aaccttgggc cttgtgctga ctgagatggg gtttggtgat tacagaattt tagcgaattt 1020
tgtaattgta cttgtttgtc tgtagttttg ttttgttttc ttgtttctca tacattcctt 1080
aggcttcaat tttattcgag tataggtcac aataggaatt caaactttga gcaggggaat 1140
taatcccttc cttcaaatcc agtttgtttg tatatatgtt taaaaaatga aacttttgct 1200
ttaaattcta ttataacttt ttttatggct gaaatttttg catgtgtctt tgctctctgt 1260
tgtaaattta ctgtttaggt actaactcta ggcttgttgt gcagtttttg aagtataacc 1320
atggtacgtc ctgtagaaac cccaacccgt gaaatcaaaa aactcgacgg cctgtgggca 1380
ttcagtctgg atcgcgaaaa ctgtggaatt gatcagcgtt ggtgggaaag cgcgttacaa 1440
gaaagccggg caattgctgt gccaggcagt tttaacgatc agttcgccga tgcagatatt 1500
cgtaattatg cgggcaacgt ctggtatcag cgcgaagtct ttataccgaa aggttgggca 1560
ggccagcgta tcgtgctgcg tttcgatgcg gtcactcatt acggcaaagt gtgggtcaat 1620
aatcaggaag tgatggagca tcagggcggc tatacgccat ttgaagccga tgtcacgccg 1680
tatgttattg ccgggaaaag tgtacgtatc accgtttgtg tgaacaacga actgaactgg 1740
cagactatcc cgccgggaat ggtgattacc gacgaaaacg gcaagaaaaa gcagtcttac 1800
ttccatgatt tctttaacta tgccggaatc catcgcagcg taatgctcta caccacgccg 1860
aacacctggg tggacgatat caccgtggtg acgcatgtcg cgcaagactg taaccacgcg 1920
tctgttgact ggcaggtggt ggccaatggt gatgtcagcg ttgaactgcg tgatgcggat 1980
caacaggtgg ttgcaactgg acaaggcact agcgggactt tgcaagtggt gaatccgcac 2040
ctctggcaac cgggtgaagg ttatctctat gaactgtgcg tcacagccaa aagccagaca 2100
gagtgtgata tctacccgct tcgcgtcggc atccggtcag tggcagtgaa gggccaacag 2160
ttcctgatta accacaaacc gttctacttt actggctttg gtcgtcatga agatgcggac 2220
ttacgtggca aaggattcga taacgtgctg atggtgcacg accacgcatt aatggactgg 2280
attggggcca actcctaccg tacctcgcat tacccttacg ctgaagagat gctcgactgg 2340
gcagatgaac atggcatcgt ggtgattgat gaaactgctg ctgtcggctt taacctctct 2400
ttaggcattg gtttcgaagc gggcaacaag ccgaaagaac tgtacagcga agaggcagtc 2460
aacggggaaa ctcagcaagc gcacttacag gcgattaaag agctgatagc gcgtgacaaa 2520
aaccacccaa gcgtggtgat gtggagtatt gccaacgaac cggatacccg tccgcaagtg 2580
cacgggaata tttcgccact ggcggaagca acgcgtaaac tcgacccgac gcgtccgatc 2640
acctgcgtca atgtaatgtt ctgcgacgct cacaccgata ccatcagcga tctctttgat 2700
gtgctgtgcc tgaaccgtta ttacggatgg tatgtccaaa gcggcgattt ggaaacggca 2760

gagaaggtac tggaaaaaga acttctggcc tggcaggaga aactgcatca gccgattatc 2820
atcaccgaat acggcgtgga tacgttagcc gggctgcact caatgtacac cgacatgtgg 2880
agtgaagagt atcagtgtgc atggctggat atgtatcacc gcgtctttga tcgcgtcagc 2940
gccgtcgtcg gtgaacaggt atggaatttc gccgattttg cgacctcgca aggcatattg 3000
cgcgttggcg gtaacaagaa agggatcttc actcgcgacc gcaaaccgaa gtcggcggct 3060
tttctgctgc aaaaacgctg gactggcatg aacttcggtg aaaaaccgca gcagggaggc 3120
aaacaatgaa tcaacaactc tcctggcgca ccatcgtcgg ctacagcctc ggtggggaat 3180
tccccggggg tacctaatag tgagatccaa cacttacgtt tgcaacgtcc aagagcaaat 3240
agaccacgna cgccggaagg ttgccgcagc gtgtggattg cgtctcaatt ctctcttgca 3300
ggaatgcaat gatgaatatg atactgacta tgaaactttg agggaatact gcctagcacc 3360
gtcacctcat aacgtgcatc atgcatgccc tgacaacatg gaacatcgct atttttctga 3420
agaattatgc tcgttggagg atgtcgcggc aattgcagct attgccaaca tcgaactacc 3480
cctcacgcat gcattcatca atattattca tgcggggaaa ggcaagatta atccaactgg 3540
caaatcatcc agcgtgattg gtaacttcag ttccagcgac ttgattcgtt ttggtgctac 3600
ccacgttttc aataaggacg agatggtgga gtaaagaagg agtgcgtcga agcagatcgt 3660
tcaaacattt ggcaataaag tttcttaaga ttgaatcctg ttgccggtct tgcgatgatt 3720
atcatataat ttctgttgaa ttacgttaag catgtaataa ttaacatgta atgcatgacg 3780
ttatttatga gatgggtttt tatgattaga gtcccgcaat tatacattta atacgcgata 3840
gaaaacaaaa tatagcgcgc aaactaggat aaattatcgc gcgcggtgtc atctatgtta 3900
ctagatcgat caaacttcgg tactgtgtaa tgacgatgag caatcgagag gctgactaac 3960
aaaaggtaca tcggtcgacg agctc 3985

<210> 19
<211> 3684
<212> DNA
<213> Artificial Sequence

<220>
<223> Description of Artificial Sequence:chimeric gene

<400> 19
   aagctttgct ctagatcaaa ctcacatcca aacataacat ggatatcttc cttaccaatc 60
   atactaatta ttttgggtta aatattaatc attattttta agatattaat taagaaatta 120
   aaagattttt taaaaaaatg tataaaatta tattattcat gatttttcat acatttgatt 180
   ttgataataa atatattttt tttaatttct taaaaaatgt tgcaagacac ttattagaca 240
   tagtcttgtt ctgtttacaa aagcattcat catttaatac attaaaaaat atttaatact 300
   aacagtagaa tcttcttgtg agtggtgtgg gagtaggcaa cctggcattg aaacgagaga 360
   aagagagtca gaaccagaag acaaataaaa agtatgcaac aaacaaatca aaatcaaagg 420
   gcaaaggctg gggttggctc aattggttgc tacattcaat tttcaactca gtcaacggtt 480
   gagattcact ctgacttccc caatctaagc cgcggatgca aacggttgaa tctaacccac 540
   aatccaatct cgttacttag ggcttttccc gtcattaact cacccctgcc acccggtttc 600
   cctataaatt ggaactcaat gctcccctct aaactcgtat cgcttcagag ttgagaccaa 660
   gacacactcg ttcatatatc tctctgctct tctcttctct tctacctctc aaggtacttt 720
   tcttctccct ctaccaaatc ctagattccg tggttcaatt tcggatcttg cacttctggt 780
   ttgctttgcc ttgctttttc ctcaactggg tccatctagg atccatgtga aactctactc 840
   tttctttaat atctgcggaa tacgcgttgg actttcagat ctagtcgaaa tcatttcata 900
   attgcctttc tttcttttag cttatgagaa ataaaatcac ttttttttta tttcaaaata 960
   aaccttgggc cttgtgctga ctgagatggg gtttggtgat tacagaattt tagcgaattt 1020
   tgtaattgta cttgtttgtc tgtagttttg ttttgttttc ttgtttctca tacattcctt 1080
   aggcttcaat tttattcgag tataggtcac aataggaatt caaactttga gcaggggaat 1140
   taatcccttc cttcaaatcc agtttgtttg tatatatgtt taaaaaatga aacttttgct 1200
   ttaaattcta ttataacttt ttttatggct gaaatttttg catgtgtctt tgctctctgt 1260
   tgtaaattta ctgtttaggt actaactcta ggcttgttgt gcagtttttg aagtataacc 1320
   atggccactt tcttcgccca aacctccttc ccctcccact ctctctccaa aaccttcgat 1380
   acccatttcg cccctgcccc gaaagtcaac gtctttgtga acttcagggc gaggaggcac 1440
   gttggggtgc gagtttcgaa cgcgctgatc gaaccagatg gagggaagct cgtggagctt 1500
   gtggtgacgg attttgagag ggatttgaag aagggtgagg ctctttcgtt gccgaggatc 1560
   aagctctcaa ggattgacct tgagtgggtc catgtcctca gcgaaggatg ggccacaccc 1620
   ctgaaaggct tcatgagaga agccgagttc ctccaaacgc ttcatttcaa ctcgctccga 1680
   ctcgatgatg ggtcggtcgt gaacatgtca gtgcccatcg tgctggctat tgatgatgcg 1740
   cagaagcatc ggatcgggga taacaaaaag gttgctcttt ttgattccaa gggagacccc 1800
   gttgcaattc tcaataatat tgagatttat aagcatccta aagaagaaag aatagcccga 1860
   acttggggaa ccattgcccc tggcctacct tatgttgaac aaactataac caatgctgga 1920
   aattggttga ttgggggtga cctagaggtc attgaaccaa ttcagtacaa tgatggactt 1980
   gatcattttc gtctatctcc ggcacaactc cgtgcagagt tcacaaggcg caatgcggat 2040
   gctgtgtttg ccttccagct ccggaatcct gttcacaatg gccatgcttt gctaatgact 2100
   gacacccgaa agcgccttct tgagatgggc tataagaatc ctgtcctctt gcttcatcca 2160
   cttggaggct acaccaaagc tgatgatgtc ccacttgatt ggcgaatgaa gcaacatgag 2220
   aaggtacttg aggatggtgt tcttgatcca gagacaactg tggtatccat attcccatct 2280
   cccatgcact atgctggacc cacggaggtg cagtggcatg caaaggctag gatcaatgca 2340
   ggggctaact tctatatcgt tggtcgtgac cccgcaggca tgagccatcc agttgagaaa 2400
   agagatctgt atgatgctga ccatggaaag aaagtattga gcatggcacc gggactagag 2460
   cgtctaaaca ttcttccttt cagggttgct gcatatgaca agactcaggg taaaatggca 2520
   ttctttgacc cttcaaggcc tcaggacttc ctgttcatat caggcacaaa gatgcgcaca 2580
   ctggcaagga acaaagaaag tcctcctgat ggatttatgt gccctggtgg atggaaggtg 2640
   ctggttgatt actatgatag cttagtactc tcaagcaacg gcaaagtgca ggaagctgtt 2700
   ccagcttaat cttgtatcat atcataatgt atatatctca tgattgggag aaaccttaag 2760
   cttatgtatt ctcctgctaa gacatacttc acgaggatcc tctggcccaa tctaataata 2820
   ataataaatt aaaactttgg ggaggcaaaa aaaaaaaaaa aaaaaaaaaa aactcgaggg 2880
   ggggcccggt acctaatagt gagatccaac acttacgttt gcaacgtcca agagcaaata 2940
   gaccacgnac gccggaaggt tgccgcagcg tgtggattgc gtctcaattc tctcttgcag 3000
   gaatgcaatg atgaatatga tactgactat gaaactttga gggaatactg cctagcaccg 3060
   tcacctcata acgtgcatca tgcatgccct gacaacatgg aacatcgcta tttttctgaa 3120
   gaattatgct cgttggagga tgtcgcggca attgcagcta ttgccaacat cgaactaccc 3180
   ctcacgcatg cattcatcaa tattattcat gcggggaaag gcaagattaa tccaactggc 3240
   aaatcatcca gcgtgattgg taacttcagt tccagcgact tgattcgttt tggtgctacc 3300
   cacgttttca ataaggacga gatggtggag taaagaagga gtgcgtcgaa gcagatcgtt 3360
   caaacatttg gcaataaagt ttcttaagat tgaatcctgt tgccggtctt gcgatgatta 3420
   tcatataatt tctgttgaat tacgttaagc atgtaataat taacatgtaa tgcatgacgt 3480
   tatttatgag atgggttttt atgattagag tcccgcaatt atacatttaa tacgcgatag 3540
   aaaacaaaat atagcgcgca aactaggata aattatcgcg cgcggtgtca tctatgttac 3600
   tagatcgatc aaacttcggt actgtgtaat gacgatgagc aatcgagagg ctgactaaca 3660
   aaaggtacat cggtcgacga gctc 3684

<210> 20
<211> 3963
<212> DNA
<213> Artificial Sequence

<220>
<223> Description of Artificial Sequence:chimeric gene

<400> 20
aagctttgct ctagatcaaa ctcacatcca aacataacat ggatatcttc cttaccaatc 60
atactaatta ttttgggtta aatattaatc attattttta agatattaat taagaaatta 120
aaagattttt taaaaaaatg tataaaatta tattattcat gatttttcat acatttgatt 180
ttgataataa atatattttt tttaatttct taaaaaatgt tgcaagacac ttattagaca 240
tagtcttgtt ctgtttacaa aagcattcat catttaatac attaaaaaat atttaatact 300
aacagtagaa tcttcttgtg agtggtgtgg gagtaggcaa cctggcattg aaacgagaga 360
aagagagtca gaaccagaag acaaataaaa agtatgcaac aaacaaatca aaatcaaagg 420
gcaaaggctg gggttggctc aattggttgc tacattcaat tttcaactca gtcaacggtt 480
gagattcact ctgacttccc caatctaagc cgcggatgca aacggttgaa tctaacccac 540
aatccaatct cgttacttag ggcttttccc gtcattaact caccccctgcc acccggtttc 600
cctataaatt ggaactcaat gctcccctct aaactcgtat cgcttcagag ttgagaccaa 660
gacacactcg ttcatatatc tctctgctct tctcttctct tctacctctc aaggtacttt 720
tcttctccct ctaccaaatc ctagattccg tggttcaatt tcggatcttg cacttctggt 780
ttgctttgcc ttgctttttc ctcaactggg tccatctagg atccatgtga aactctactc 840
tttctttaat atctgcggaa tacgcgttgg actttcagat ctagtcgaaa tcatttcata 900
attgcctttc tttcttttag cttatgagaa ataaaatcac ttttttttta tttcaaaata 960
aaccttgggc cttgtgctga ctgagatggg gtttggtgat tacagaattt tagcgaattt 1020

tgtaattgta cttgtttgtc tgtagttttg ttttgttttc ttgtttctca tacattcctt 1080
aggcttcaat tttattcgag tataggtcac aataggaatt caaactttga gcaggggaat 1140
taatcccttc cttcaaatcc agtttgtttg tatatatgtt taaaaaatga aacttttgct 1200
ttaaattcta ttataacttt ttttatggct gaaatttttg catgtgtctt tgctctctgt 1260
tgtaaattta ctgtttaggt actaactcta ggcttgttgt gcagtttttg aagtataacc 1320
atggccgttt cgagctcgca catgcgtttc acctttgagt gccgctccga tcccgatttc 1380
tcgccccccc cgccgtcctt cgacaacctc cgccgccgaa acttccgctc ctccgcagga 1440
tccggcgcgg cgtttcacgg catctcctcc ctcatcctcc gcttccctcc caacttccag 1500
cgccagctaa gcaccaaggc gcgccgcaac tgcagcaaca tcggcgtcgc gcaaatcgtc 1560
gccgcttcgt ggtcgaacaa cagcgacaac tctccggccg ccggggctcc ggcgccgccc 1620
gcggccaccg ccacggacgc cgctacggtg cctctccccg tcgtcgtcgc cgccaacgag 1680
gacgtcgttg tctccgccgc ggcagacgag aacggggctg tacagttaaa cagtagttct 1740
tattcttcat ttttgaaatc cgatgcaagc aaaacgattc atgccgctga aagactgggt 1800
aggggtattg agactgatgg aattaccacc cctgtggtta acacttctgc ctactttttt 1860
aagaaaaccg ctgatctcat tgatttcaag gagaatcgtc aagtgagtta tgaatacggg 1920
cgctatggaa acccaacgac ggtggttctg gaggagaaga taagtgcatt ggagggggcc 1980
gaatcaactg tgataatggc gtctgggatg tgtgctagcg tagtcctgtt tatggcactg 2040
gttccagctg gtggacatct tgtgaccact acggattgtt ataggaagac tagaatattc 2100
attgagactt ttcttccaaa gatggggatc acgaccactg taattgatcc agcagatgtt 2160
ggagccttgg aatctgcatt ggagcagcac aatgtgtctc tattcttcac tgagtctcct 2220
accaatccat tcctgagatg tgttgatatt aagctggttt cagagctttg ccacaagaag 2280
gggactttgc tctgtattga tggtacattt gcaactccat tgaaccagaa ggcccttgcc 2340
cttggcgctg atctgattct gcactcctta acaaaataca tgggtggaca tcatgatgtc 2400
cttggtggtt gcataagtgg ttcaattaag gtggtttcgc aaattcggac tttgcaccat 2460
gttttgggtg gtacacttaa cccgaatgct gcatacctat tcatcagagg catgaaaacg 2520
ctgcatctcc gtgtacagca gcagaattca acaggaatga ggatggccaa acttttagag 2580
gcacatccca aggtgaagcg ggtctactat ccaggcttgc cgagtcaccc tgaacatgag 2640
cttgccaaga ggcagatgac tggtttcggt ggtgttgtca gttttgagat tgatggagat 2700
ctacatacca caataaaatt tattgattca ttgaaaatcc catatattgc ggcctcgttt 2760
ggtggctgtg agagcattgt ggatcaacct gctattttgt cttactggga tcttcctcag 2820
tcagaaaggg ccaagtacaa gatttatgac aacctggttc gcttcagctt tggagttgaa 2880
gattttgagg atttgaaggc tgatgtcctg caagctctgg aagctatata gacagttttc 2940
ctgattcacc caagtttttt tcttttataa ttgtgctatt tgtttgttat cacatctggc 3000
gattcaattg aattttgatc gtctaatgtt ctgttggaat tgtgttaaga tgaatggtct 3060
ctaatttgga tgttatgaaa cttgtgatga attgttgaaa ttgaaacctc tatttgatga 3120
aaaaaaaaaa aaaaaaaaaa aaaaaaaaaa actcgagggg gggcccggta cctaatagtg 3180
agatccaaca cttacgtttg caacgtccaa gagcaaatag accacgnacg ccggaaggtt 3240
gccgcagcgt gtggattgcg tctcaattct ctcttgcagg aatgcaatga tgaatatgat 3300
actgactatg aaactttgag ggaatactgc ctagcaccgt cacctcataa cgtgcatcat 3360
gcatgccctg acaacatgga acatcgctat ttttctgaag aattatgctc gttggaggat 3420
gtcgcggcaa ttgcagctat tgccaacatc gaactacccc tcacgcatgc attcatcaat 3480
attattcatg cggggaaagg caagattaat ccaactggca aatcatccag cgtgattggt 3540
aacttcagtt ccagcgactt gattcgtttt ggtgctaccc acgttttcaa taaggacgag 3600
atggtggagt aaagaaggag tgcgtcgaag cagatcgttc aaacatttgg caataaagtt 3660
tcttaagatt gaatcctgtt gccggtcttg cgatgattat catataattt ctgttgaatt 3720
acgttaagca tgtaataatt aacatgtaat gcatgacgtt atttatgaga tgggttttta 3780
tgattagagt cccgcaatta tacatttaat acgcgataga aaacaaaata tagcgcgcaa 3840
actaggataa attatcgcgc gcggtgtcat ctatgttact agatcgatca aacttcggta 3900
ctgtgtaatg acgatgagca atcgagaggc tgactaacaa aaggtacatc ggtcgacgag 3960
ctc 3963

## Description

### FIELD OF THE INVENTION

This invention relates to a plant promoter, in particular, to an S-adenosyl-L-methionine synthetase (SAMS) promoter and subfragments thereof and their use in regulating the expression of at least one heterologous nucleic acid fragment in plants.

### BACKGROUND OF THE INVENTION

Recent advances in plant genetic engineering have opened new doors to engineer plants having improved characteristics or traits, such as, resistance to plant diseases, insect resistance, herbicidal resistance, enhanced stability or shelf-life of the ultimate consumer product obtained from the plants and improvement of the nutritional quality of the edible portions of the plant. Thus, a desired gene (or genes) from a source different than the plant, but engineered to impart different or improved characteristics or qualities, can be incorporated into the plant's genome. This new gene (or genes) can then be expressed in the plant cell to exhibit the new trait or characteristic.

In order to obtain expression of the newly inserted gene in the plant cell, the proper regulatory signals must be present and be in the proper location with respect to the gene. These regulatory signals include a promoter region, a 5' non-translated leader sequence and a 3' transcription termination/polyadenylation sequence.

A promoter is a DNA sequence that directs cellular machinery of a plant to produce RNA from the contiguous coding sequence downstream (3') of the promoter. The promoter region influences the rate, developmental stage, and cell type in which the RNA transcript of the gene is made. The RNA transcript is processed to produce messenger RNA (mRNA) which serves as a template for translation of the RNA sequence into the amino acid sequence of the encoded polypeptide. The 5' non-translated leader sequence is a region of the mRNA upstream of the protein coding region that may play a role in initiation and translation of the mRNA. The 3' transcription termination/polyadenylation signal is a non-translated region downstream of the protein coding region that functions in the plant cells to cause termination of the RNA transcript and the addition of polyadenylate nucleotides to the 3' end of the RNA.

It has been shown that certain promoters are able to direct RNA synthesis at a higher rate than others. These are called "strong promoters". Certain other promoters have been shown to direct RNA production at higher levels only in particular types of cells or tissues and are often referred to as "tissue specific promoters". In this group, many seed storage protein genes' promoters have been well characterized and widely used, such as the phaseolin gene promoter of *Phaseolus vulgaris,* the helianthinin gene of sunflower, the β-conglycinin gene of soybean (Chen et al., (1989) Dev. Genet. 10, 112-122), the napin gene promoter of *Brassica napus* (Ellerstrom et al, (1996) Plant Mol. Biol. 32, 1019-1027), the oleosin gene promoters of *Brassica* and *Arabidopsis* (Keddie et al, (1994) Plant Mol. Biol. 24, 327-340; Li, (1997) Texas A&M Ph.D. dissertation, pp. 107-128; Plant et al, (1994) Plant Mol. Biol. 25, 193-20S). Another class of tissue specific promoters is described in, U.S. Patent No. 5,589,583, issued to Klee et al. on December 31, 1996; these plant promoters are capable of conferring high levels of transcription of chimeric genes in meristematic tissues and/or rapidly dividing cells. In contrast to tissue-specific promoters, "inducible promoters" direct RNA production in response to certain environmental factors, such as heat shock, light, hormones, ion concentrations etc. (Espartero et al, (1994) Plant Mol. Biol. 25, 217-227; Gomez-Gomez and Carrasco, (1998) Plant Physiol. 117, 397-405; Holtorf et al, (1995) Plant Mol. Biol. 29, 637-646; MacDowell et al, (1996) Plant Physiol.111, 699-711; Mathur et al, (1992) Biochem. Biophys. Acta 1137, 338-348; Mett et al, (1996) Transgenic Res. 5, 105-113; Schoffl et al, (1989) Mol. Gen. Genet. 217,246-253; Ulmasov et al, (1995) Plant Physiol. 108, 919-927*).*

Promoters that are capable of directing RNA production in many or all tissues of a plant are called "constitutive promoters". The ideal constitutive promoter should be able to drive gene expression in all cells of the organism throughout its development. Expression of many so-called constitutive genes, such as actin (McDowell et al., (1996) Plant Physiol. 111, 699-711; Wang et al., (1992) Mol. Cell Biol. 12, 3399-3406), and ubiquitin (Callis et al, (1990) J Biol. Chem. 265, 12486-12493; Rollfinke et al, (1998) Gene 211, 267-276) varies depending on the tissue types and developmental stages of the plant. The most widely used constitutive promoter, the cauliflower mosaic virus 3SS promoter, also shows variations in activity in different plants and in different tissues of the same plant (Atanassova et al., (1998) Plant Mol. Biol. 37,275-285; Battraw and Hall, (1990) Plant Mol. Biol. 15, 527-538 ; Holtorf et al., (1995) Plant Mol. Biol. 29, 637-646; Jefferson et al., (1987) EMBO J. 6, 3901-3907; Wilmink et al., (1995) Plant Mol. Biol. 28, 949-955). The cauliflower mosaic virus 35S promoter is also described in U.S. Patent No. 5,106,739. The tissue-specific expression and synergistic interactions of sub-domains of the promoter of cauliflower mosaic virus are discussed in U.S. Patent No. 5,097,025, which issued to Benfey et al. on March 17, 1992. A *Brassica* promoter (hsp80) that provides for constitutive expression of heterologous genes in a wide range of tissues and organs is discussed in U.S. Patent No. 5,612,472 which issued to Wilson et al. on March 18, 1997.

Since the patterns of expression of a chimeric gene (or genes) introduced into a plant are controlled using promoters, there is an ongoing interest in the isolation and identification of novel promoters which are capable of controlling expression of a chimeric gene or (genes).

### SUMMARY OF THE INVENTION

This invention concerns an isolated nucleic acid fragment comprising a promoter wherein said promoter consists essentially of the nucleotide sequence set forth in SEQ ID NOs:6, 14, 15, or 16 or said promoter consists essentially of a fragment or subfragment that is substantially similar and functionally equivalent to the nucleotide sequence set forth in SEQ ID NOs:6, 14, 15, or 16.

In a second embodiment, this invention concerns a chimeric gene comprising at least one heterologous nucleic acid fragment operably linked to the promoter of the invention.

In a third embodiment, this invention concerns plants containing this chimeric gene and seeds obtained from such plants.

In a fourth embodiment, this invention concerns a method of increasing or decreasing the expression of at least one heterologous nucleic acid fragment in a plant cell which comprises:
(a) transforming a plant cell with the chimeric gene described above;
(b) growing fertile mature plants from the transformed plant cell of step (a);
(c) selecting plants containing the transformed plant cell wherein the expression of the heterologous nucleic acid fragment is increased or decreased.

In a fifth embodiment, this invention concerns an isolated nucleic acid fragment comprising a constitutive plant SAMS promoter.

### BRIEF DESCRIPTION OF THE DRAWINGS AND SEQUENCES

The invention can be more fully understood from the following detailed description, the drawings and the Sequence Descriptions that form a part of this application. The Sequence Descriptions contain the three letter codes for amino acids as defined in 37 C.F.R. §§ 1.821-1.825, which are incorporated herein by reference.

SEQ ID NO: is the nucleotide sequence comprising the entire cDNA insert in clone s2.12b06 which encodes a soybean S-adenosyl-L-methionine synthetase protein.

SEQ ID NO:2 is the nucleotide sequence comprising a soybean S-adenosyl-L-methionine synthetase genomic DNA fragment.

SEQ ID NO:3 is the nucleotide sequence of a portion of the cDNA insert in clone srrlc.pk002.b21 encoding a portion of a soybean S-adenosyl-L-methionine synthetase protein.

SEQ ID NO:4 is a 32 base oligonucleotide primer, designated sam-5, used to amplify the soybean S-adenosyl-L-methionine synthetase promoter region via PCR.

SEQ ID NO:5 is a 24 base oligonucleotide primer, designated sam-6, used to amplify the soybean S-adenosyl-L-methionine synthetase promoter region via PCR.

SEQ ID NO:6 is the nucleotide sequence comprising a soybean S-adenosyl-L-methionine synthetase promoter fragment produced via PCR using primers sam-5 (SEQ ID NO:4) and sam-6 (SEQ ID NO:5).

SEQ ID NO:7 is a 22 base oligonucleotide primer, designated sam-9, used to amplify the soybean S-adenosyl-L-methionine synthetase promoter region via PCR.

SEQ ID NO:8 is a 19 base oligonucleotide primer, designated atps-9, used to amplify a chimeric gene comprising a SAMS promoter fragment and a portion of the ATP sulfurylase (ATPS) gene via PCR.

SEQ ID NO:9 is a 21 base oligonucleotide primer, designated cgs-8, used to amplify a chimeric gene comprising a SAMS promoter and a portion of the cystathionine-γ-synthase 1 (CGS1) gene via PCR.

SEQ ID NO: 1 0 is a 20 base oligonucleotide antisense primer, designated atps-4, used to amplify the ATP sulfurylase transcript via RT-PCR.

SEQ ID NO: I is a 21 base oligonucleotide antisense primer, designated cgs-10, used to amplify the cystathionine-y-synthase 1 transcript via RT-PCR.

SEQ ID NO: 12 is a 20 base oligonucleotide primer, designated atps-3, used to amplify an ATP sulfurylase cDNA via PCR.

SEQ ID NO: 13 is a 23 base oligonucleotide primer, designated cgs-9, used to amplify a cystathionine-γ-synthase 1 cDNA via PCR.

SEQ ID NO: 14 is a 2165 nucleotide sequence comprising a soybean S-adenosyl-L-methionine synthetase genomic DNA fragment which starts at the 5' end of SEQ ID NO:2, and ends at the ATG translation start codon of the S-adenosyl-L-methionine synthetase.

SEQ ID NO: 15 is a 1574 nucleotide sequence comprising a DNA fragment which starts at the 5' end of SEQ ID NO:2, and ends at the ATG translation start codon of the S-adenosyl-L-methionine synthetase, and wherein a 591 nucleotide intron sequence has been removed.

SEQ ID NO:16 is a 719 nucleotide sequence comprising a DNA fragment which starts at nucleotide 4 of SEQ ID NO:6, and ends at the ATG translation start codon of the S-adenosyl-L-methionine synthetase, and wherein a 591 nucleotide intron sequence has been removed.

SEQ ID NO: 17 is a 6975 nucleotide sequence comprising plasmid pMH40Δ.

SEQ ID NO: 18 is a 3985 nucleotide sequence comprising a SAMS promoter::GUS::3' Nos DNA fragment present in plasmid PZSL11

SEQ ID NO: 19 is a 3684 nucleotide sequence comprising a SAMS promoter::ATPS::3' Nos DNA fragment.

SEQ ID NO:20 is a 3963 nucleotide sequence comprising a SAMS promoter::CGS1::3' Nos DNA fragment.

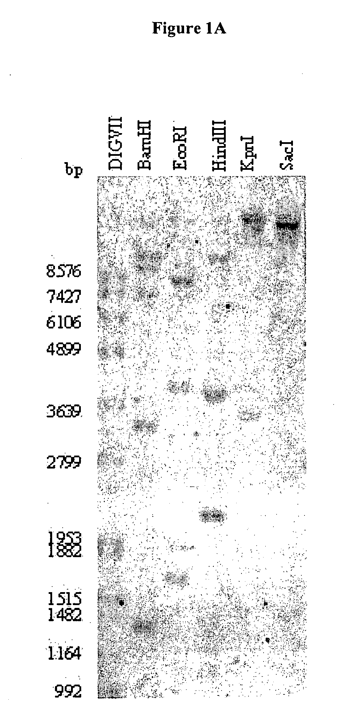

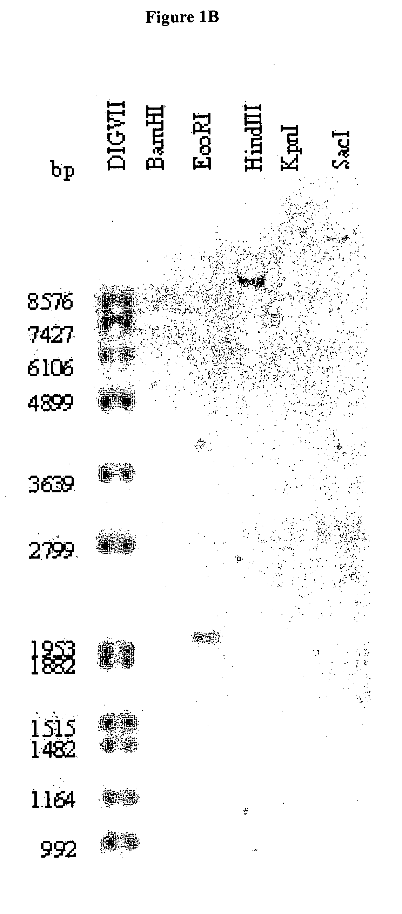

Figures 1A and 1B depict Southern hybridization analyses of SAMS genes. Soybean genomic DNA was digested with BamHI, EcoRI, HindIII, KpnI, and SacI , and then the blot was hybridized with a full length SAMS cDNA (SEQ ID NO:1) probe in Figure 1A or with a SAMS promoter fragment (SEQ ID NO:6) probe inFigure 1B.

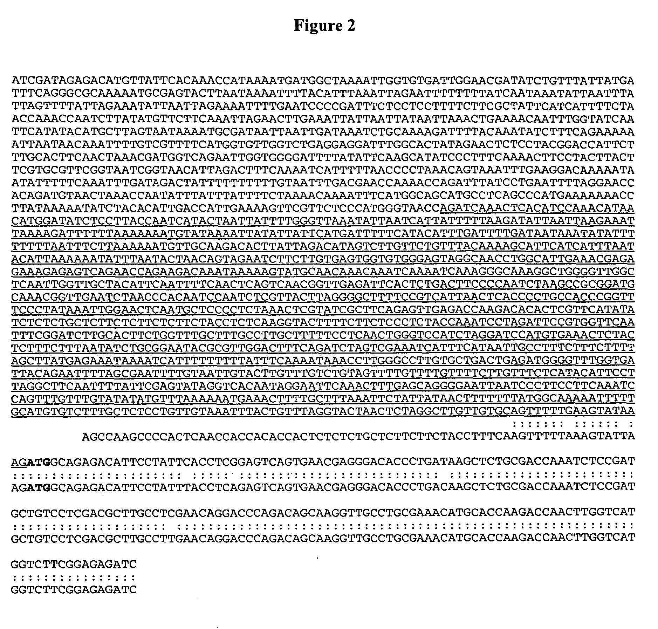

Figure 2 depicts a SAMS genomic DNA sequence (SEQ ID NO:2) and the alignment of the overlapping region with SAMS cDNA sequence (SEQ ID NO:1). The 2336 bp SAMS genomic DNA sequence has a 191 bp region aligned with the 5' end sequence of the SAMS cDNA with six mismatches. The region used to make the SAMS promoter by adding the Ncol site at its 3' end is underlined. The translation start codon is in bold.

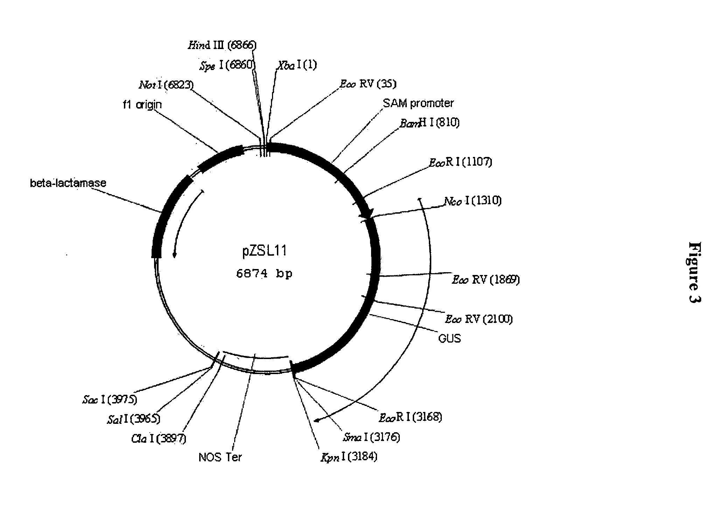

Figure 3 depicts the structure of the SAMS::GUS expression cassette. The SAMS promoter was cloned into pMH40Δ to replace its 35S promoter. The structure of the resulted SAMS::GUS construct was generated by Vector NTI^{™} software (InforMax, Inc., North Bethesda, MD).

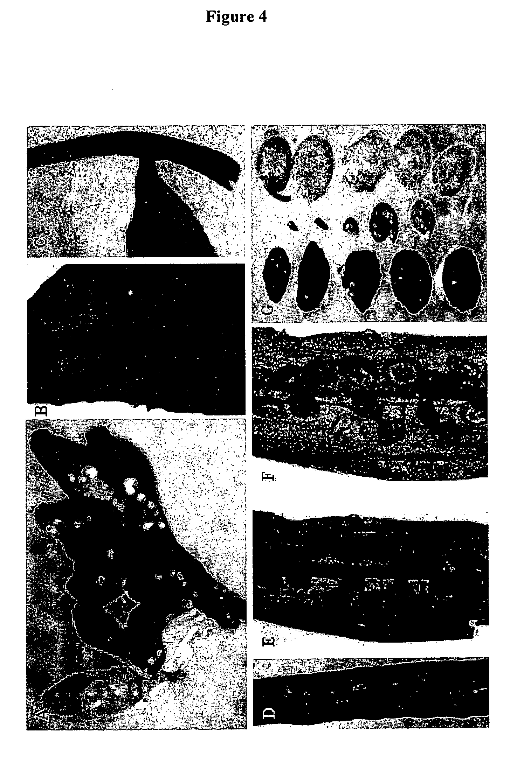

Figure 4 depicts a histochemical GUS expression analysis of transgenic *Arabidopsis* plants harboring the SAMS::GUS expression cassette. *Arabidopsis* tissues were incubated at 37°C with X-Gluc overnight and dehydrated with ethanol. (A) Flower buds; (B) leaf; (C) Inflorescence stem and a cauline leaf; (D, E, F) developing siliques; (G) Developing seeds and embryos. All of the seeds were derived from GUS-positive siliques. Genetic segregation of the GUS gene was demonstrated by the blue funiculus of the white seed in the right upper corner.

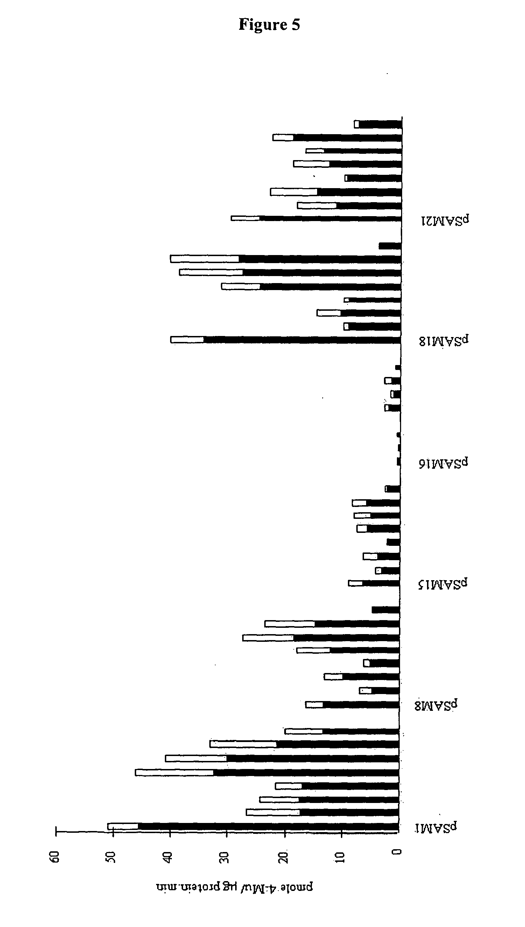

Figure 5 depicts a fluorometric GUS expression assay of transgenic *Arabidopsis* plants harboring the SAMS::GUS expression cassette. Triple samples of flowers, leaves, stems, siliques coats, young seeds, medium seeds, old seeds, and dry seeds collected from SAMS::GUS transgenic *Arabidopsis* plants were assayed for GUS activity. The graph was generated by Microsoft Excel and the standard deviation is indicated by the upper part of each column.

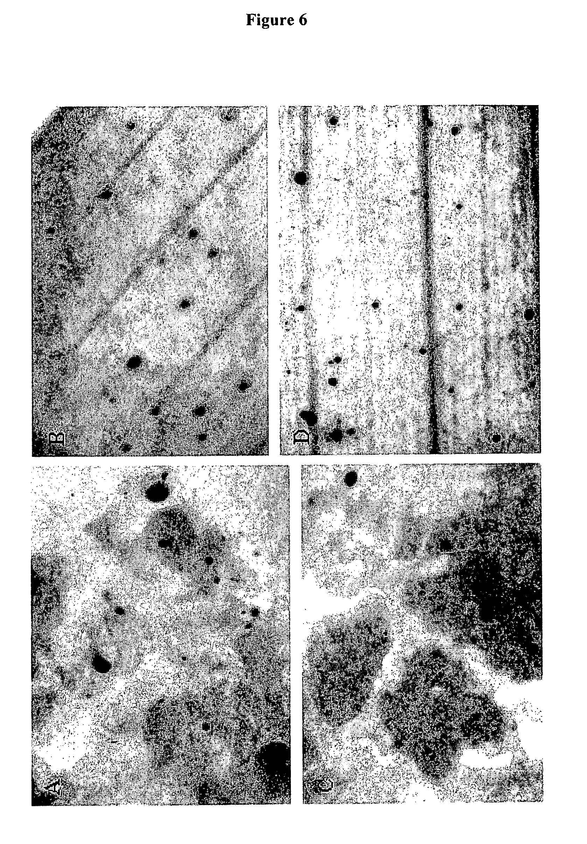

Figure 6 depicts a histochemical GUS transient expression analysis of SAMS promoter in corn. The pZSL1 1 (SAMS::GUS) or the pMH40Δ (35S::GUS) plasmid DNA was delivered into corn callus (A, C) or leaf discs (B, D), and the GUS activity was detected by incubation with X-Gluc overnight at 37° C. (A, B) Transformed with pZSL1 1 DNA; (C, D) Transformed with pMH40Δ DNA.

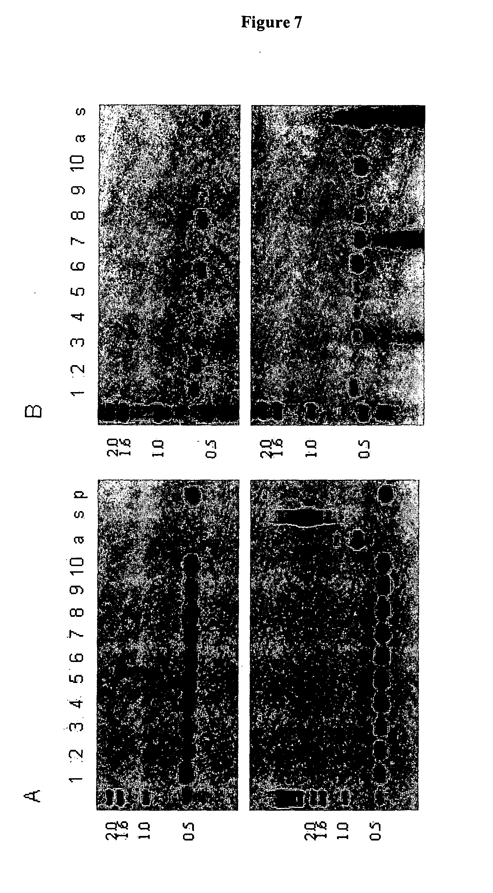

Figures 7(A) and 7(B) depict the presence and expression of transgenic soybean ATPS and CGS 1 genes controlled by the SAMS promoter in transgenic *Arabidopsis* plants. Figure 7(A) is a PCR analysis. Genomic DNA of ten transgenic *Arabidopsis* plants (1 to 10), wild type *Arabidopsis* (a), wild type soybean (s), and plasmid DNA of SAMS::CGS 1 or SAMS::ATPS in binary vectors (p) were used as templates in PCR with gene-specific primers. PCR of ten SAMS::CGS 1 transgenic plants with primer sam-9 which is specific to SAMS promoter, and primer cgs-8 which is specific to soybean CGS 1 (upper). PCR of ten SAMS::ATPS transgenic plants with primer sam-9 which is specific to SAMS promoter, and primer atps-1 which is specific to soybean ATPS gene (lower). Figure 7(B) is an RT-PCR analysis. Total leaf RNA of ten transgenic *Arabidopsis* plants (1 to 10), wild type *Arabidopsis* (a), and wild type soybean (s) were used as templates in RT-PCR with gene-specific primers. First strand cDNA was synthesized from a gene-specific antisense primer with reverse transcriptase, and then the first strand cDNA was amplified by PCR with both sense and antisense primers. RT-PCR of ten SAMS::CGS 1 transgenic plants with primers, cgs-9 (sense) and cgs-10 (antisense), specific to soybean CGS 1 gene (upper). RT-PCR of ten SAMS::ATPS transgenic plants with primers, atps-3 (sense) and atps-4 (antisense), specific to soybean ATPS gene (lower).

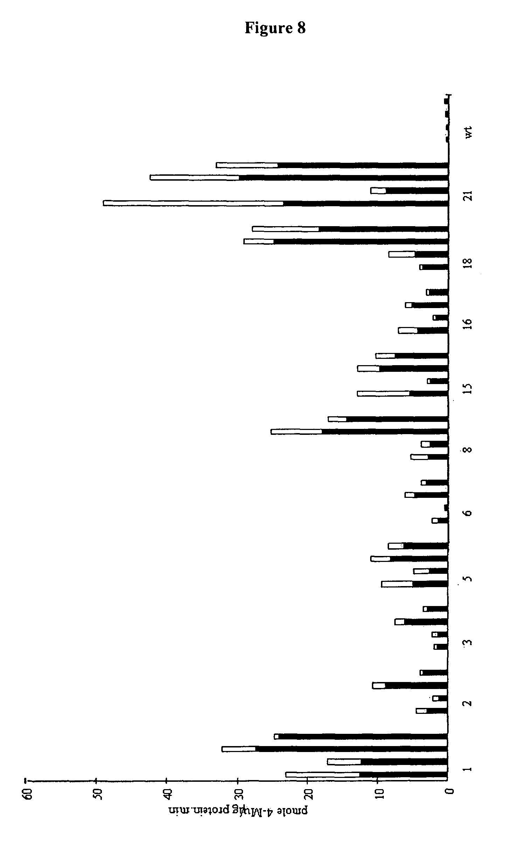

Figure 8 depicts induction of SAMS promoter activity by methionine. Seeds of ten transgenic Arabidopsis lines transformed with SAMS::GUS construct were germinated on filter papers soaked with H₂O 1x Murashige and Skoog salt, 0.01 mM, and 1 mM methionine. Ten days old seedlings were harvested and assayed for GUS activity. The solid bar and hollow bar indicate, respectively, the average and the standard variation of three samples for each treatment.

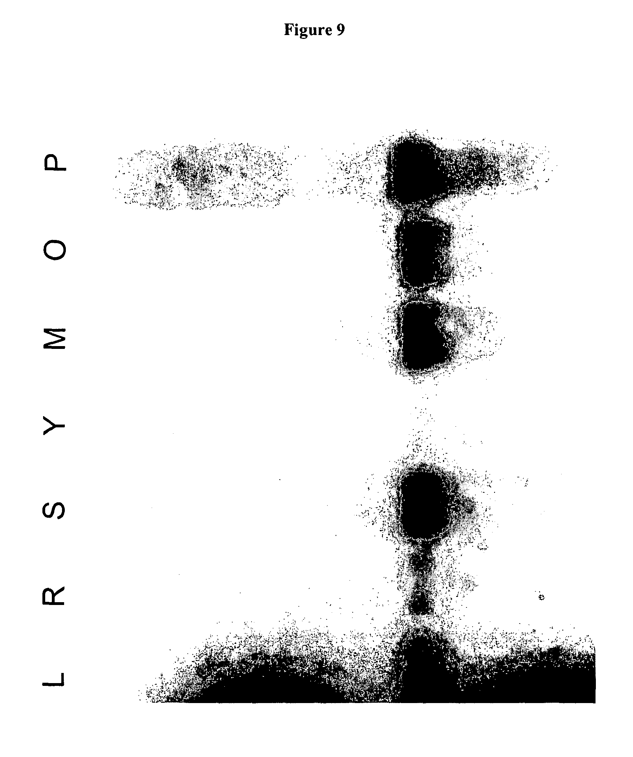

Figure 9 depicts a northern hybridization. Soybean total RNAs from leaves, roots, stems, young seeds, medium seeds, old seeds, and pod coats (L, R, S, Y, M, O, and P) were used to make the RNA blot which was hybridized with a full length SAMS cDNA (SEQ ID NO:1) probe.

### DETAILED DESCRIPTION OF THE INVENTION

In the context of this disclosure, a number of terms shall be utilized.

As used herein, an "isolated nucleic acid fragment" is a polymer of ribonucleotides (RNA) or deoxyribonucleotides (DNA) that is single- or double-stranded, optionally containing synthetic, non-natural or altered nucleotide bases. An isolated nucleic acid fragment in the form of DNA may be comprised of one or more segments of cDNA, genomic DNA or synthetic DNA.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of chimeric genes to produce the desired phenotype in a transformed plant. Chimeric genes can be designed for use in co-suppression or antisense by linking a nucleic acid fragment or subfragment thereof, whether or not it encodes an active enzyme, in the appropropriate orientation relative to a plant promoter sequence.

The terms "substantially similar" and "corresponding substantially" as used herein refer to nucleic acid fragments wherein changes in one or more nucleotide bases does not affect the ability of the nucleic acid fragment to mediate gene expression or produce a certain phenotype. These terms also refer to modifications of the nucleic acid fragments of the instant invention such as deletion or insertion of one or more nucleotides that do not substantially alter the functional properties of the resulting nucleic acid fragment relative to the initial, unmodified fragment. It is therefore understood, as those skilled in the art will appreciate, that the invention encompasses more than the specific exemplary sequences.

Moreover, the skilled artisan recognizes that substantially similar nucleic acid sequences encompassed by this invention are also defined by their ability to hybridize, under moderately stringent conditions (for example, 0.5 X SSC, 0.1% SDS, 60° C) with the sequences exemplified herein, or to any portion of the nucleotide sequences reported herein and which are functionally equivalent to the promoter of the invention. Preferred substantially similar nucleic acid sequences encompassed by this invention are those sequences that are 80% identical to the nucleic acid fragments reported herein or which are 80% identical to any portion of the nucleotide sequences reported herein. More preferred are nucleic acid fragments which are 90% identical to the nucleic acid sequences reported herein, or which are 90% identical to any portion of the nucleotide sequences reported herein. Most preferred are nucleic acid fragments which are 95% identical to the nucleic acid sequences reported herein, or which are 95% identical to any portion of the nucleotide sequences reported herein. Sequence alignments and percent similarity calculations may be determined using the Megalign program of the LASARGENE bioinformatics computing suite (DNASTAR Inc., Madison, WI). Multiple alignment of the sequences are performed using the Clustal method of alignment (Higgins and Sharp (1989) CABIOS. 5:151-153) with the default parameters (GAP PENALTY=10, GAP LENGTH PENALTY=10). Default parameters for pairwise alignments and calculation of percent identiy of protein sequences using the Clustal method are KTUPLE=1, GAP PENALTY=3, WINDOW=5 and DIAGONALS SAVED=5. For nucleic acids these parameters are GAP PENALTY=10, GAP LENGTH PENALTY=10, KTUPLE=2, GAP PENALTY=5, WINDOW=4 and DIAGONALS SAVED=4. A "substantial portion" of an amino acid or nucleotide sequence comprises enough of the amino acid sequence of a polypeptide or the nucleotide sequence of a gene to afford putative identification of that polypeptide or gene, either by manual evaluation of the sequence by one skilled in the art, or by computer-automated sequence comparison and identification using algorithms such as BLAST (Altschul, S. F., et al., (1993) J. Mol. Biol. 215:403-410) and Gapped Blast (Altschul, S. F. et al., (1997) Nucleic Acids Res. 25:3389-3402); see also www.ncbi.nlm.nih.gov/BLAST/).

"Gene" refers to a nucleic acid fragment that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

A "heterologous nucleic acid fragment" refers to a nucleic acid fragment comprising a nucleic acid sequence that is different from the nucleic acid sequence comprising the plant promoter of the invention.

"Coding sequence" refers to a DNA sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences.

"Promoter" refers to a DNA sequence capable of controlling the expression of a coding sequence or functional RNA. The promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue-specificity of a promoter. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. Promoters which cause a gene to be expressed in most cell types at most times are commonly referred to as "constitutive promoters". New promoters of various types useful in plant cells are constantly being discovered; numerous examples may be found in the compilation by Okamuro and Goldberg (1989, Biochemistry of Plants 15:1-82). It is further recognized that since in most cases the exact boundaries of regulatory sequences have not been completely defined, DNA fragments of some variation may have identical promoter activity. An "intron" is an intervening sequence in a gene that is transcribed into RNA but is then excised in the process of generating the mature mRNA. The term is also used for the excised RNA sequences. An "exon" is a portion of the sequence of a gene that is transcribed and is found in the mature messenger RNA derived from the gene, but is not necessarily a part of the sequence that encodes the final gene product.

The "translation leader sequence" refers to a DNA sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner, R. and Foster, G. D. (1995) Molecular Biotechnology 3:225).

The "3' non-coding sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) Plant Cell 1:671-680.

"RNA transcript" refers to a product resulting from RNA polymerase-catalyzed transcription of a DNA sequence. When an RNA transcript is a perfect complementary copy of a DNA sequence, it is referred to as a primary transcript or it may be a RNA sequence derived from posttranscriptional processing of a primary transcript and is referred to as a mature RNA. "Messenger RNA" ("mRNA") refers to RNA that is without introns and that can be translated into protein by the cell. "cDNA" refers to a DNA that is complementary to and synthesized from an mRNA template using the enzyme reverse transcriptase. The cDNA can be single-stranded or converted into the double-stranded by using the klenow fragment of DNA polymerase I. "Sense" RNA refers to RNA transcript that includes mRNA and so can be translated into protein within a cell or *in vitro.* "Antisense RNA" refers to a RNA transcript that is complementary to all or part of a target primary transcript or mRNA and that blocks expression or transcripts accumulation of a target gene (U.S. Patent No. 5,107,065). The complementarity of an antisense RNA may be with any part of the specific gene transcript, i.e. at the 5' non-coding sequence, 3' non-coding sequence, introns, or the coding sequence. "Functional RNA" refers to antisense RNA, ribozyme RNA, or other RNA that may not be translated but yet has an effect on cellular processes.

"Sense" RNA refers to RNA transcript that includes the mRNA and so can be translated into protein by the cell. "Antisense RNA" refers to a RNA transcript that is complementary to all or part of a target primary transcript or mRNA and that blocks the expression of a target gene (U.S. Patent No. 5,107,065. The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes.

The term "operably linked" refers to the association of nucleic acid sequences on a single nucleic acid fragment so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

The term "expression", as used herein, refers to the production of a functional end-product. Expression or overexpression of a gene involves transcription of the gene and translation of the mRNA into a precursor or mature protein. "Antisense inhibition" refers to the production of antisense RNA transcripts capable of suppressing the expression of the target protein. "Overexpression" refers to the production of a gene product in transgenic organisms that exceeds levels of production in normal or non-transformed organisms. "Co-suppression" refers to the production of sense RNA transcripts capable of suppressing the expression or transcript accumulation of identical or substantially similar foreign or endogenous genes (U.S. Patent No. 5,231,020). The mechanism of co-suppression may be at the DNA level (such as DNA methylation), at the transcriptional level, or at posttranscriptional level.

"Altered expression" refers to the production of gene product(s) in transgenic organisms in amounts or proportions that differ significantly from the amount of the gene product(s) produced by the corresponding wild-type organisms.

"Transformation" refers to the transfer of a nucleic acid fragment into the genome of a host organism, resulting in genetically stable inheritance. Host organisms containing the transformed nucleic acid fragments are referred to as "transgenic" organisms. The preferred method of corn cell transformation is use of particle-accelerated or "gene gun" transformation technology (Klein et al. (1987) Nature (London) 327:70-73; U.S. Patent No. 4,945,050).

Standard recombinant DNA and molecular cloning techniques used herein are well known in the art and are described more fully in Sambrook, J., Fritsch, E. F. and Maniatis, T., Molecular Cloning: A Laboratory Manual; Cold Spring Harbor Laboratory Press, Cold Spring Harbor, 1989 (hereinafter "Sambrook et al., 1989") or Ausubel, F. M., Brent, R., Kingston, R. E., Moore, D. D., Seidman, J. G., Smith, J. A. and Struhl, K. (eds.), Current Protocols in Molecular Biology , John Wiley and Sons, New York, 1990 (hereinafter "Ausubel et al., 1990").

"PCR" or "Polymerase Chain Reaction" is a technique for the synthesis of large quantities of specific DNA segments, consists of a series of repetitive cycles (Perkin Elmer Cetus Instruments, Norwalk, CT). Typically, the double stranded DNA is heat denatured, the two primers complementary to the 3' boundaries of the target segment are annealed at low temperature and then extended at an intermediate temperature. One set of these three consecutive steps comprises a cycle.

An "expression construct" is a plasmid vector or a subfragment thereof comprising the instant chimeric gene. The choice of plasmid vector is dependent upon the method that will be used to transform host plants. The skilled artisan is well aware of the genetic elements that must be present on the plasmid vector in order to successfully transform, select and propagate host cells containing the chimeric gene. The skilled artisan will also recognize that different independent transformation events will result in different levels and patterns of expression (Jones et al., (1985) EMBO J 4:2411-2418; De Almeida et al.,(1989) Mol. Gen. Genetics 218:78-86), and thus that multiple events must be screened in order to obtain lines displaying the desired expression level and pattern. Such screening may be accomplished by Southern analysis of DNA, Northern analysis of mRNA expression, Western analysis of protein expression, or phenotypic analysis.

Although the SAMS enzyme is present in most plant cell types, no SAMS promoter capable of driving gene expression in most or all plant cell types has been described. Previous studies indicated that plants contain multiple SAMS genes which are differentially expressed in response to various stresses (Schroder et al. (1997) Plant Mol. Biol. 33:211-222). A SAMS promoter that is preferentially active in a particular tissue type, i.e. vascular (Peleman et al., (1989) Plant Cell 1, 81-93; Mijnsbrugge et al., (1996) Plant Cell Physiol. 37,1108-1115), was also known. However, it was not possible to predict, before the studies reported herein, whether any SAMS gene was controlled by a constitutive promoter. It is demonstrated herein that constitutive SAMS promoters do, in fact, exist in plants, and that such promoters can be readily isolated and used by one skilled in the art.

This invention concerns an isolated nucleic acid fragment comprising a constitutive plant SAMS promoter. This invention also concerns an isolated nucleic acid fragment comprising a promoter wherein said promoter consists essentially of the nucleotide sequence set forth in SEQ ID NOs:6, 14, 15 or 16 or said promoter consists essentially of a fragment or subfragment that is substantially similar and functionally equivalent to the nucleotide sequence set forth in SEQ ID NOs:6, 14, 15 or 16. A nucleic acid fragment that is functionally equivalent to the instant SAMS promoter is any nucleic acid fragment that is capable of controlling the expression of a coding sequence or functional RNA in a similar manner to the SAMS promoter. The expression patterns of the SAMS promoter are defined in the following paragraphs.

Northern-blot hybridization experiments indicated that SAMS gene transcripts are present in a variety of soybean tissues and that the abundance of SAMS gene transcripts does not differ greatly from tissue to tissue (Figure 9 and Example 3). Strong expression of the SAMS gene was also inferred by the high frequency of occurrences of cDNA sequences with homology to SAMS (ESTs) in a soybean cDNA sequence database created by sequencing random cDNAs from libraries prepared from many different soybean tissues. ESTs encoding SAMS can be easily identified by conducting BLAST (Basic Local Alignment Search Tool; Altschul, S. F., et al., (1993) J. Mol. Biol. 215:403-410; see also www.ncbi.nlm.nih.gov/BLAST/) searches for similarity to sequences contained in the BLAST "nr" database, e.g., SAMS from Oryza sativa (EMBL Accession No. Z26867) or SEQ ID NO:1 provided herein. SAMS homologs were among the most abundant classes of cDNAs found in the soybean libraries. This indicated that SAMS was a highly expressed gene in most soybean cell types. The data obtained from sequencing many SAMS ESTs also indicated that there were several SAMS isoforms encoded by the soybean genome.

A soybean cDNA clone designated s2.12b06 was found to encode a protein which is very similar to the protein encoded by the cDNA to *Oryza sativa* SAMS (pLog value for this match was 61.59). The soybean cDNA clone designated s2.12b06 was completely sequenced (SEQ ID NO:1) and found to contain an opening reading frame which encodes a full length SAMS polypeptide. Southern hybridization analysis of soybean genomic DNA with this full length SAMS cDNA as a probe suggested that there are approximately four related SAMS genes in the soybean genome (Figure 1 A), which is consistent with the EST sequencing data.

The soybean SAMS cDNA clone was used to isolate a soybean genomic DNA fragment containing more than 2000 nucleotides upstream (5') of the SAMS protein coding sequence by hybridization of a soybean genomic DNA library to the SAMS cDNA fragment probe. Southern hybridization analysis of soybean genomic DNA using a 1314 base pair DNA fragment from upstream of the SAMS protein coding sequence as a probe indicated that this fragment is unique in the soybean genome (Figure 1B).

The promoter activity of the soybean genomic DNA fragment upstream of the SAMS protein coding sequence was assessed by linking the fragment to a reporter gene, the *E*. *coli* β-glucuronidase gene (GUS) (Jefferson (1987) Plant Mol. Biol. Rep. 5:387-405), transforming the SAMS promoter::GUS expression cassette into *Arabidopsis,* and analyzing GUS expression in various cell types of the transgenic plants. GUS expression was detected in all parts of the transgenic plants that were analyzed. These results indicated that the nucleic acid fragment contained a constitutive promoter. Since SAMS catalyzes the reaction to synthesize S-adenosyl-L-methionine from methionine and ATP, free methionine levels might regulate SAMS promoter activity. To see if the SAMS promoter is regulated by external methionine, the SAMS::GUS transgenic Arabidopsis seeds were germinated in the presence or absence of methionine. Ten day old seedlings were analyzed for GUS activity according to the protocol described in Example 5. Ten independent transgenic lines were tested and all of them responded similarly. GUS activity was more than two-fold higher in seedlings germinated in the presence of methionine (Figure 8). The increased SAMS promoter activity in the presence of methionine may be particularly useful for efforts to increase methionine biosynthesis via overexpression of enzymes in the methionine biosynthetic pathway or the sulfate assimilation pathway. It is clear from the disclosure set forth herein that one of ordinary skill in the art could readily isolate a constitutive plant SAMS promoter from any plant by performing the following procedure:
1) obtaining a SAMS cDNA from a desired plant by any of a variety of methods well known to those skilled in the art including, but not limited to, (a) random sequencing of ESTs from a cDNA library and characterizing the ESTs via a BLAST search as described above; or (b) hybridizing a cDNA library to a known plant SAMS cDNA; or (c) PCR amplification using oligonucleotide primers designed from known SAMS cDNAs;
2) obtaining a genomic DNA fragment that includes approximately 500 to 3000 nucleotides from the region 5' to a SAMS protein coding sequence, which contains a SAMS promoter, by hybridization of a genomic DNA library to a SAMS cDNA fragment probe;
3) operably linking the nucleic acid fragment containing the region upstream (5') of the SAMS protein coding sequence to a suitable reporter gene ; there are a variety of reporter genes that are well known to those skilled in the art, including the bacterial GUS gene, the firefly luciferase gene, and the green fluorescent protein gene; any gene for which an easy an reliable assay is available can serve as the reporter gene
4) transforming a chimeric SAMS promoter::reporter gene expression cassette into an appropriate plant for expression of the promoter. There are a variety of appropriate plants which can be used as a host for transformation that are well known to those skilled in the art, including the dicots, *Arabidopsis,* tobacco, soybean, oilseed rape, peanut, sunflower, safflower, cotton, tomato, potato, cocoa and the monocots, corn, wheat, rice, barley and palm. The terms "oilseed rape" and "oilseed Brassica" are used interchangeably herein.
5) testing for expression of a SAMS promoter in various cell typesof transgenic plants, e.g., leaves, roots, flowers, seeds, transformed with the chimeric SAMS promoter::reporter gene expression cassette by assaying for expression of the reporter gene product. A constitutive SAMS promoter will produce high level expression of the reporter in all, or nearly all, of the plant tissues tested.

In another aspect, this invention concerns a chimeric gene comprising at least one heterologous nucleic acid fragment operably linked to the promoter of the present invention. Chimeric genes can be constructed by operably linking the nucleic acid fragment of the invention, i.e., the SAMS promoter or a fragment or a subfragment that is substantially similar and functionally equivalent to any portion of the nucleotide sequence set forth in SEQ ID NOS:6, 14, 15 or 16, to a heterologous nucleic acid fragment. Any heterologous nucleic acid fragment can be used to practice the invention. The selection will depend upon the desired application or phenotype to be achieved. The various nucleic acid sequences can be manipulated so as to provide for the nucleic acid sequences in the proper orientation.

Plasmid vectors comprising the instant chimeric genes can then be constructed. The choice of plasmid vector is dependent upon the method that will be used to transform host cells. The skilled artisan is well aware of the genetic elements that must be present on the plasmid vector in order to successfully transform, select and propagate host cells containing the chimeric gene.

The plasmid vectors or chimeric genes can be used to transform plant cells. Transformation techniques are well known to those skilled in art as discussed above. A preferred method of plant cell transformation is the use of particle-accelerated or "gene gun" transformation technology (Klein et al. (1978) Nature (London) 327:70-73; U.S. Patent No. 4,945,050). The chimeric gene will normally be joined to a marker for selection in plant cells. The marker may be resistance to a biocide, particularly an antibiotic, such as kanamycin, G418, bleomycin, hygromycin, chloramphenicol, or the like. The particular marker employed will be one which will allow for selection of transformed cells as compared to cells lacking the heterologous nucleic acid sequence which has been introduced. Examples of plant cells which can be transformed using plant transformation techniques include, but are not limited to, monocot and dicot plant cells such as soybean, oilseed *Brassica* species, corn, peanut, rice, wheat, sunflower, safflower, cotton,cocoa,tobacco,tomato, potato, barley, palm, Arabidopsis and the like.

In addition to the bacterial GUS gene, two soybean genes, ATP sulfurylase (ATPS) and cystathionine-β-synthase 1 (CGS1), were also successfully expressed by this promoter in transgenic Arabidopsis, as depicted in Figure 7. This further validates the application of the SAMS promoter of the invention in plant genetic engineering practice.

The skilled artisan will also recognize that different independent transformation events will result in different levels and patterns of expression of the chimeric genes (Jones et al., (1985) EMBO J. 4:2411-2418; De Almeida et al., (1989) Mol. Gen. Genetics 218:78-86). Thus, multiple events must be screened in order to obtain lines displaying the desired expression level and pattern. Such screening may be accomplished by northern analysis of mRNA expression, western analysis of protein expression, or phenotypic analysis. Also of interest are seeds obtained from transformed plants displaying the desired expression profile.

The level of activity of the SAMS promoter is comparable to that of many known strong promoters, such as the CaMV 35S promoter (Atanassova et al., (1998) Plant Mol. Biol. 37:275-285; Battraw and Hall, (1990) Plant Mol. Biol. 15:527-538; Holtorf et al., (1995) Plant Mol. Biol. 29:637-646; Jefferson et al., (1987) EMBO J 6:3901-3907; Wilmink et al., (1995) Plant Mol. Biol. 28:949-955), the *Arabidopsis* oleosin promoters (Plant et al., (1994) Plant Mol. Biol. 25:193-205; Li, (1997) Texas A&M University Ph.D. dissertation, pp. 107-128), the *Arabidopsis* ubiquitin extension protein promoters (Callis *et al.,* 1990), a tomato ubiquitin gene promoter (Rollfinke *et al.,* 1998), a soybean heat shock protein promoter (Schoffl *et al.,* 1989), and a maize H3 histone gene promoter (Atanassova *et al.,* 1998).

Expression of the chimeric genes in most plant cell makes the SAMS promoter of the instant invention especially useful when constitutive expression of a target heterologous nucleic acid fragment is required. Examples of suitable target heterologous nucleic acid fragments include, but are not limited to, a herbicide-resistance or pathogen-resistance nucleic acid fragment. Another useful feature of the constitutive plant SAMS promoter is its expression profile in developing seeds. The SAMS promoter of the invention is most active in developing seeds at early stages and gradually turns down at later stages. Such activity is indicated by the GUS activity detected in seeds of transgenic *Arabidopsis* plants containing a SAMS::GUS expression cassette as shown in Figures 4 and 5. The expression profile of the claimed SAMS promoter is different from that of many seed-specific promoters, e.g., seed storage protein promoters, which often provide highest activity in later stages of development (Chen et al., (1989) Dev. Genet. 70.112-122; Ellerstrom et al., (1996) Plant Mol. Biol. 32:1019-1027; Keddie et al., (1994) Plant Mol. Biol. 24:327-340; Plant et al., (1994) Plant Mol. Biol. 25:193-205; Li, (1997) Texas A&M University Ph.D. dissertation, pp. 107-128). Thus, the SAMS promoter will be a very attractive candidate when overexpression of a gene in embryos is desired at an early developing stage. For example, it may be desirable to overexpress a gene regulating early embryo development or a gene involved in the metabolism prior to seed maturation.

One general application of the SAMS promoter of the invention is to construct chimeric genes that can be used in the selection of transgenic cell lines in plant transformation. Currently, many of the selectable marker genes for plant transformation are under the control of the cauliflower mosaic virus 35S promoter. Since the SAMS promoter of the invention is active in seedlings and callus, the appropriate selection phase for transgenic plants or cell lines, this promoter may be used as an alternative to the 35S promoter to drive the expression of selectable marker genes.

Another general application of the SAMS promoter of the invention is to construct chimeric genes that can be used to reduce expression of at least one heterologous nucleic acid fragment in a plant cell. To accomplish this a chimeric gene designed for cosuppression of a heterologous nucleic acid fragment can be constructed by linking the fragment to the SAMS promoter of the present invention. (See U.S. Patent No. 5,231,020 for methodology to block plant gene expression via cosuppression.) Alternatively, a chimeric gene designed to express antisense RNA for a heterologous nucleic acid fragment can be constructed by linking the fragment in reverse orientation to the SAMS promoter of the present invention. (See U.S. Patent No. 5,107,065 for methodology to block plant gene expression via antisense RNA.) Either the cosuppression or antisense chimeric gene can be introduced into plants via transformation. Transformants wherein expression of the heterologous nucleic acid fragment is decreased or eliminated are then selected.

This invention also concerns a method of increasing or decreasing the expression of at least one heterologous nucleic acid fragment in a plant cell which comprises:
(a) transforming a plant cell with the chimeric genes described herein;
(b) growing fertile mature plants from the transformed plant cell of step (a);
(c) selecting plants containing a transformed plant cell wherein the expression of the heterologous nucleic acid fragment is increased or decreased.

Transformation and selection can be accomplished using methods well-known to those skilled in the art including, but not limited to, the methods described herein.

### EXAMPLES

The present invention is further defined in the following Examples. From the above discussion and these Examples, one skilled in the art can ascertain the essential characteristics of this invention, and without departing from the spirit and scope thereof, can make various changes and modifications of the invention to adapt it to various usages and conditions.

Unless otherwise stated, all parts and percentages are by weight and degrees are Celsius. Techniques in molecular biology were typically performed as described in Ausubel, F. M., et al., (1990, Current Protocols in Molecular Biology, John Wiley and Sons, New York) or Sambrook, J. et al., (1989, Molecular cloning - A Laboratory Manual, 2nd ed. Cold Spring Harbor Laboratory Press, Cold Spring Harbor, New York).

### EXAMPLE 1

### Composition of cDNA Libraries, Isolation and Sequencing of cDNA Clones

cDNA libraries representing mRNAs from soybean tissues were prepared in Uni-ZAP XR^{™} vectors according to the manufacturer's protocol (Stratagene, La Jolla, CA). Conversion of the Uni-ZAP XR^{™} libraries into plasmid libraries was accomplished according to the protocol provided by Stratagene. Upon conversion, cDNA inserts were contained in the plasmid vector pBluescript^{™} (Stratagene). DNA was prepared for sequencing from randomly selected bacterial colonies containing recombinant pBluescript^{™} plasmids either by amplifying the cDNA inserts via polymerase chain reaction using primers specific for vector sequences flanking the cloning site or by preparing plasmid DNA from cultured bacterial cells. Amplified insert DNAs or plasmid DNAs were sequenced in dye-primer sequencing reactions using a Perkin Elmer Model 377 fluorescent sequencer to generate partial cDNA sequences termed expressed sequence tags or "ESTs" (see Adams, M. D. et al., (1991) Science 252:1651).

### EXAMPLE 2

### Identification of SAMS cDNA Clones

ESTs encoding SAMS were identified by conducting BLAST (Basic Local Alignment Search Tool; Altschul, S. F., et al., (1993) J. Mol. Biol. 215:403-410; see also www.ncbi.nlm.nih.gov/BLAST/) searches for similarity to sequences contained in the BLAST "nr" database (comprising all non-redundant GenBank CDS translations, sequences derived from the 3-dimensional structure Brookhaven Protein Data Bank, the last major release of the SWISS-PROT protein sequence database, EMBL, and DDBJ databases). The cDNA sequences obtained in Example 1 were analyzed for similarity to all publicly available DNA sequences contained in the "nr" database using the BLASTN algorithm provided by the National Center for Biotechnology Information (NCBI). The DNA sequences were translated in all reading frames and compared for similarity to all publicly available protein sequences contained in the "nr" database using the BLASTX algorithm (Gish, W. and States, D. J. (1993) Nature Genetics 3:266-272 and Altschul, S. F., et al. (1997) Nucleic Acids Res. 25:3389-3402) provided by the NCBI. For convenience, the P-value (probability) of observing a match of a cDNA sequence to a sequence contained in the searched databases merely by chance as calculated by BLAST are reported herein as "pLog" values, which represent the negative of the logarithm of the reported P-value. Accordingly, the greater the pLog value, the greater the likelihood that the cDNA sequence and the BLAST "hit" represent homologous proteins.

The BLASTX search using the nucleotide sequence from clone s2.12b06 revealed that this nucleotide sequence encoded a protein that was similar to the protein encoded by the cDNA to *Oryza sativa* (EMBL Accession No. Z26867) S-adenosylmethionine synthetase; the pLog value for this match was 61.59. This cDNA clone was completely sequenced (SEQ ID NO:1) and found to contain an opening reading frame ranging from nucleotides 74 to 1252 which is predicted to encode a full length SAMS polypeptide.

A high level of expression of the SAMS genes was inferred by the high frequency of occurrences of soybean cDNA sequences with homology to *Oryza sativa* SAMS obtained from many different cDNA libraries prepared from many different soybean cell types. SAMS homologs were the third most abundant class of ESTs found in the soybean libraries. Although the ranking might not represent a precise estimate of the relative abundance of the SAMS transcripts *in vivo* in all soybean libraries, due to the selective use of different cDNA libraries, it did indicate that SAMS was a highly expressed gene. The EST sequence data also revealed that there were several SAMS isoforms in the soybean genome.

### EXAMPLE 3

### S-adenosylmethionine Synthetase is Encoded by a Gene Family

Southern hybridization analysis of soybean genomic DNA with a full length SAMS cDNA (SEQ ID NO:1) as a probe suggested that there are at least four related SAMS genes in the soybean genome (Figure 1 A). The DNA probe for Southern hybridization was prepared as follows: plasmid DNA was prepared from an overnight bacteria culture in LB broth (GIBCO BRL, Gaithersburg, MD) using QIAprep^{™} miniprep kit (Qiagen, Valencia, CA); cDNA inserts encoding SAMS were excised by restriction enzyme digestion and recovered from agarose gel following electrophoretic separation using QIAquick^{™} gel extraction kit (Qiagen). The 1518 bp SAMS cDNA fragment (SEQ ID NO:1) was labeled with digoxigenin-dUTP as a probe by random primed DNA labeling (Boehringer Mannheim). Twenty micrograms of soybean geneomic DNA was digested with different restriction enzymes and the resulted fragments were resolved on a 0.7% agarose gel. The DNA gel was depurinated in 0.25 M HCl, denatured in 0.5 M NaOH/1.5 M NaCl, neutralized in 1 m Tris-Cl, pH 8.0/1.5 M NaCl, and transferred in 20x SSC (GIBCO BRL) to nylon membrane (Boehringer Mannheim). The Southern blot was hybridized with the SAMS cDNA-specific probe at 45°C overnight in Easy Hyb (Boehringer Mannheim). The blot was washed 10 minutes in 2xSSC/0.1% SDS, and 3x 10 minutes in 0.1 x SSC/0.1 % SDS at 65°C. The hybridized probe was detected with chemiluminescent reagent CDP-Star (Boehringer Mannheim) according to the manufacturer's protocol. Multiple bands were detected in BamHI, EcoRI, and HindIII digestions (Figure 1A). The large band in KpnI and SacI digestions may represent more than one DNA fragment because the band is too big for good resolution. The hybridization patterns presented in Figure 1A and the analysis of partial SAMS cDNA sequences from DuPont's EST database suggest that there are at least four copies of the SAMS gene in the soybean genome and that their sequences are conserved.

The 1314 bp SAMS promoter fragment (SEQ ID NO:6) was labeled with digoxigenin-dUTP also by random primed DNA labeling (Boehringer Mannheim). The labeled SAMS promoter probe was used to hybridize the same Southern blot as above described. The SAMS promoter-specific probe hybridized to a single band in each of the five different digestions, BamHI, EcoRI, HindIII, KpnI, and SacI (Figure 1B). The results indicate that the SAMS promoter has only a single copy in soybean genome.

A northern hybridization experiment indicated that SAMS gene transcripts were present in a variety of soybean tissues and that the abundance of SAMS gene transcripts did not differ greatly from tissue to tissue. Total RNAs were extracted from soybean leaves, stems, young seeds, medium seeds, old seeds, and pod coats using Trizol^{™} Reagent according to the manufacturer's protocol (GIBCO BRL). Ten micrograms of total RNA were loaded in each well of a 1.2% agarose gel containing 7% fonnaldehyde in 1x MOPS buffer, 20 mM 3-[N-morpholino]propane-sulfonic acid, 5 mM sodium acetate, 1 mM EDTA, pH 6.0. RNA was transferred to nylon filters (Micron Separations Inc., Westborough, MA) in 10X SSC and crosslinked to the filters with UV light. Filters were hybridized with probes prepared from cDNA insert fragments in 50% deionized formamide, 5x SSPE, 1 x Denhardt's solution, 0.1% SDS, and 100 µg denatured salmon sperm DNA (Sigma, St. Louis, MO) at 42° for 24 hours. Filters were washed in 2x SSPE and 0.1% SDS at room temperature for 10 minutes, 1x SSPE and 0.1% SDS at 65° for 10 minutes, and then in 0.1x SSPE and 0.1% SDS at 65° for 10 minutes. Filters were exposed to Kodak X-ray film at -80. The abundance of SAMS transcripts in leaves, roots, stems, young seeds, medium seeds, old seeds, and pod coats can be seen in Figure 9. The weak signals observed in the hybridizations to RNA samples from root and young seed were attributed to underloading, because hybridizations with ribosomal RNAs that serve as internal controls were also relatively weak in those samples (data not shown). Because of the high sequence similarities among the four SAMS gene isoforms, this RNA gel blot was not able to indicate how the isoforms were distributed in any particular tissue. However, the experiment demonstrated that all examined soybean tissues contained SAMS messenger RNA.

### EXAMPLE 4

### Cloning of the Soybean S-adenosylmethionine Synthetase Gene Promoter

The soybean full length SAMS cDNA (SEQ ID NO: 1), obtained in Example 2, was used to generate a probe to isolate a SAMS promoter. The full length SAMS cDNA sequence consisted of 1518 bp, and it had a 74 bp 5'-untranslated region and a PstI site at position 296. Because the cDNA clone was harbored in a pBluescript^{™} SK vector having a PstI site upstream of the EcoRI cloning site, digestion of the clone with Pst1 generated a 315 bp fragment of DNA. The resulting restriction fragment contained 19 bp of vector and cloning linker adapter sequence in addition to the 296 bp of SAMS cDNA sequence. This PstI fragment was labeled with α-³²P-dCTP, as described in Example 3, and used as a probe to screen a soybean genomic DNA library that had been constructed in a EMBL3 SP6/T7 vector (ClonTech, Palo Alto, CA). The library was plated with LE392 (ClonTech) cells at 50,000 plaque forming units (pfu) per 150 mm NZCYM agar plate (GIBCO BRL). Plaques were transferred to Hybond nylon membranes, and the plaque replicas were then denatured and neutralized according to the manufacturer (Amersham Life Science, Arlington Heights, IL). The phage DNA was fixed on the membranes by UV-crosslinking (Stratagene). After prehybridization at 65° for 1 hour in 0.5 M NaHPO₄ pH 7.2, 1 mM EDTA, 1% crystalline BSA (Sigma), and 7% SDS, the SAMS 315 5 bp Pst1 fragment probe was denatured in boiling water bath for 5 minutes and added to the same hybridization solution, and was hybridized at 65° for 24 hours. The membranes were washed in 40 mM NaHPO₄ pH 7.2, 1 mM EDTA, 0.5% crystalline BSA, and 5% SDS for 10 minutes at room temperature, and then 3x 10 minutes at 65° in 40 mM NaHPO₄ pH 7.2, 1 mM EDTA, and 1% SDS. The membranes were exposed to Kodak X-ray film (Sigma) at -80°. Positive SAMS genomic DNA phage clones were suspended in SM buffer, 50 mM Tris-Cl, pH 7.5, 100 mM NaCl, 0.2% MgSO₄•7H₂O, and 0.1% gelatin, and purified by a secondary screening following the same procedure. Twenty three strongly hybridizing plaques were identified by the first screening from a total of 3x10⁵ pfu, and fifteen were later purified. DNAs were prepared from two of the purified phage clones (Ausubel et al., (1990) pp. 1.13.4-1.13.8), they were digested with BamHI, CIaI, PstI, and NcoI and prepared for a Southern blot. The blot was hybridized with the SAMS 315 bp PstI fragment probe prepared and used as above. A single positive fragment of clone 1 was identified from the ClaI digestion. Since the ClaI restriction site in the cDNA clone is 843 bp from the 5' end of the full length cDNA, the 2.5 kb ClaI fragment was expected to include about 1.7 kb of DNA upstream of the coding sequence, which was considered sufficient to contain the SAMS promoter.

The 2.5 kb ClaI genomic DNA fragment was cloned into pBluescript^{™} KS and the DNA insert was sequenced. The 3' end sequence of the genomic DNA fragment was expected to match the 5' end sequence of SAMS cDNA from the 5' end to the CIaI site at position 843. However, comparison of the genomic DNA sequence and the cDNA sequence revealed that the two sequences have 191 bp of overlapping sequence starting at position 54 and ending at position 245 of the cDNA sequence (SEQ ID NO: 1). The sequence of the 2.5 kb genomic DNA clone downstream of the 191 bp overlapping region was determined to be derived from the cloning vector, lambda EMBL3 SP6/T7, which contributed 257 bp of sequence to the 3' end of the 2.5 kb SAMS ClaI fragment including the ClaI cloning site. Therefore, the soybean derived DNA in the 2.5 kb ClaI fragment is described by the 2336 bp DNA sequence shown in SEQ ID NO:2.

The DNA sequence of the genomic DNA in the 191 bp region (from nucleotide 2145 to the end of the sequence) was very similar to, but did not match perfectly, the cDNA sequence; there were six base pair mismatches in this region. This was not surprising, because it was known from the experiments described in Example 3 that there is a small family of SAMS genes in soybean. It was concluded that this genomic clone is not derived from the same gene from which the cDNA used as the probe was transcribed. It was also noted that the 53 bp at the 5' end of the cDNA did not show any similarity to the genomic sequence upstream of the 191 bp overlapping region (Figure 2).

A BLASTN search of the DuPont soybean EST database using the nucleotide sequence from the soybean SAMS genomic DNA upstream of the 191 bp region revealed many cDNA clones that matched a 60 bp region of the genomic DNA from nucleotide 1496 to 1555. The sequence of one such cDNA, designated srrlc.pk002.b21, is shown in SEQ ID NO:3.

The cDNA sequence in SEQ ID NO:3 perfectly matches the genomic sequence in SEQ ID NO:2 from nucleotide 2 to 60 of the cDNA. There follows a region of 591 nucleotides in the genomic DNA that is absent from the cDNA. Then the region from nucleotide 60 to 250 of the cDNA perfectly matches the 191 bp region at the 3' end of the genomic DNA. This indicates the presence of a 591 nucleotide intron in the genomic DNA in the 5' transcribed, but untranslated, region of the SAMS gene. The presence of consensus 5' and 3' splice junctions in the genomic DNA at the exon-intron junctions supports this conclusion. Thus, the 53 bp at the 5' end of the cDNA used as the probe (SEQ ID NO:1) did not match the genomic sequence because the genomic sequence at that position in the alignment was from the intron. However, the 53 bp at the 5' end of the cDNA of SEQ ID N0:1 is very similar to the 60 nucleotides at the 5' end of the cDNA of SEQ ID NO: 3, suggesting that the gene from which SEQ ID NO:1 was transcribed also contains an intron at the analogous position.

A 1305 bp SAMS genomic DNA fragment starting at nucleotide 856 and ending at nucleotide 2160 of SEQ ID NO:2: was amplified by PCR from the 2.5 kb CIaI clone. The promoter fragment was amplified from this fragment using primers sam-5 (SEQ ID NO:4) and sam-6 (SEQ ID NO:5) and Pfu DNA polymerase (Stratagene).

| | |
|---|---|
| CATGCCATGGCTTTATACTTCAAAAACTGCAC | (SEQ ID NO:4) |
| GCTCTAGATCAAACTCACATCCAA | (SEQ ID NO:5) |

An XbaI site and an NcoI site were introduced to the 5' end and 3' end, respectively, of the PCR fragment by using these specifically designed primers. The NcoI site includes the ATG start codon of the SAMS coding region. The resulting 1314 bp fragment is shown in SEQ ID NO:6 and includes the SAMS promoter and the translation leader region, which is interrupted by the 591 nucleotide intron.

Using PCR amplification procedures and appropriate primers additional SAMS promoter fragments can be produced from the 2336 nucleotide fragment of SEQ ID NO:2. These include, but are not limited to, the three fragments provided in SEQ ID NOs:l4, 15 and 16. SEQ ID NO: 14 is a 2165 nucleotide sequence of a SAMS promoter DNA fragment which starts at the 5' end of the 2336 nucleotide sequence of SEQ ID NO:2 and ends at the ATG translation start codon of the SAMS protein. SEQ ID NO: 15 is a 1574 nucleotide sequence of a SAMS promoter DNA fragment which starts at the 5' end of the 2336 nucleotide sequence of SEQ ID NO:2 and ends at the ATG translation start codon of the SAMS protein, and from which the 591 nucleotide long intron sequence has been removed. SEQ ID NO: 16 is a 719 nucleotide sequence of a SAMS promoter DNA fragment which starts at nucleotide 4 of SEQ ID NO:6 and ends at the ATG translation start codon of the SAMS protein, and from which the 591 nucleotide long intron sequence has been removed.

### EXAMPLE 5

### Expression of the GUS Gene by the SAMS Promoter in Arabidopsis

The activity of the soybean SAMS promoter was tested by its ability to express the GUS reporter gene in transgenic *Arabidopsis* plants carrying the SAMS promoter::GUS::3' Nos expression casstette. GUS refers to the *E*. *coli* β-glucuronidase gene (GUS) (Jefferson, (1987) Plant Mol. Biol. Rep. 5:387-405) and 3' Nos refers to the transcription termination region from the nopaline synthase (Nos) gene (Depicker et al. (1982) J. Mol. Appl. Genet. 1:561-570). The SAMS promoter fragment (SEQ ID NO:6) was digested with XbaI and NcoI and inserted into plasmid pMH40Δ (SEQ ID NO: 17), which contained a 35S promoter::GUS::3' Nos plant expression cassette. The XbaI/NcoI SAMS promoter DNA fragment replaced the 35S promoter of pMH40Δ, to form the pZSL1 plasmid (Figure 3). The SAMS promoter::GUS::3' Nos DNA fragment (SEQ ID NO:18) was excised from pZSL1 by HindIII and SacI digestion and transferred into the corresponding sites of pBI101 (ClonTech) binary vector. The cloned SAMS promoter was sequenced to verify that no sequence error was generated by the PCR amplification.

The SAMS::GUS expression cassette was introduced into wild type *Arabidopsis thaliana* by *Agrobacteria* mediated transformation. *A. thaliana* ecotype columbia were grown in 228 chamber with continuous light and transformed by vacuum infiltration method using GV3101 *Agrobacteria* (Bent, A. et al., (1994) Science 265:1856-1860). Transformed *Arabidopsis* seeds were selected by germination on Murashige and Skoog minimal salt (GIBCO BRL) plus 0.2 % phytagel (Sigma), 1% sucrose, and 100 mg/ml kanamycin. The kanamycin resistant seedlings were transferred into soil and grown in 228 chamber under continuous light.

For histochemical GUS staining, plant tissues were incubated in 0.5% 5-bromo-4-chloro-3-indoxyl-β-D-glucuronic acid (X gluc, Biosynth AG, Switzerland) in 50 mM sodium phosphate, pH 7.0, 10 mM EDTA, 0.5 mM potassium ferricyanide, and 0.5 mM potassium ferrocyanide at 378 overnight, and then chlorophyll was removed with 75% ethanol. Pictures were taken using a Nikon dissecting microscope. Strong GUS expression was detected in all the parts of the transgenic *Arabidopsis* plants, including flowers (Figure 4A), leaves (Figure 4B), stems (bolt) (Figure 4C), silique coats and developing seeds (Figure 4D-F), developing embryos (Figure 4G), and seedlings (not shown). The GUS staining on leaves and silique coats was uniform with all the veins and mesophyll tissues similarly stained, while staining on flowers and stems was not uniform. Although some seeds were not stained for GUS activity due to genetic segregation, the funiculi that connected these seeds to the silique coat stained positively for GUS activity (Figure 4G). These results indicated that the soybean SAMS promoter was a constitutive promoter and was able to function in heterologous plant.

The GUS activities of the transgenic *Arabidopsis* plants were further analyzed by a fluorometric assay. For fluorescence analysis, plant tissues were ground in microfuge tubes with extraction buffer, 50 mM phosphate buffer, pH 7.0, 10 mM EDTA, 0.1% Triton X-100, 0.1% N-lauroyl sarcosine, and 10 mM β-mercaptoethanol, to homogeneity. The samples were centrifuged at 14,000 rpm for 10 minutes, and aliquots of the supernatant were used to determine protein concentrations by the Bradford method (Bio-Rad, Hercules, CA) using 96 well microtiter plates read with a kinetic microplate reader (Molecular Devices, Sunnyvale, CA). The β-glucuronidase activities were analyzed by standard protocol (Jefferson et al, (1987) EMBO J. 6:3901-3907) using 96 well microtiter plates read with Cytofluor multiwell plate reader (PerSeptive Biosystems, Framingham, MA). Data were entered into a Microsoft Excel spread sheet and analyzed. Triple samples of flower, leaf, stem, silique coat, young seed (white), medium seed (light green), old seed (dark green), and dry seed from six plants were analyzed. The soybean SAMS promoter was active in all the tissues analyzed (Figure 5). Promoter activity varied among the six lines, as is typically seen among plant transformants. The basic expression patterns were similar among all the lines, and the average SAMS promoter activity was comparable to that of the 35S promoter (Battraw and Hall, (1990) Plant Mol. Biol. 15:527-538; Jefferson et al.' (1987) EMBO J. 6:3901-3907; Atanassova et al., (1998) Plant Mol. Biol. 37:275-285; Holtorf et al., (1995) Plant Mol. Biol. 29:637-646; Wilmink et al., (1995) Plant Mol. Biol. 28:949-955). The SAMS promoter was very active in developing seeds, especially in early and medium stages of development, and the GUS specific activities are in the range of 5-40 pmole 4-Mu (4-methylumbelliferone) per microgram protein per minute, which are comparable to many strong promoters (Atanassova et al., (1998) Plant Mol. Biol. 37:275-285; Comai et al., (1990) Plant Mol. Biol. 15:373-381; Holtorf et al., (1995) Plant Mol. Biol. 29:637-646; Wilmink et al., (1995) Plant Mol. Biol. 28:949-955).

### EXAMPLE 6

### Expression of GUS Gene by SAMS Promoter in Corn

In order to test whether the dicot SAMS promoter also worked in monocot plants, pZSL 11 was introduced into corn leaf discs and callus by gene bombardment for transient gene expression assay using the biolistic particle delivery system PDS-1000/He (Bio Rad, Hercules, CA). The pMH40Δ plasmid DNA (as set forth in SEQ ID NO: 17), which contained the 35S promoter and GUS reporter gene, was also introduced into corn callus and leaf discs by gene bombardment to serve as a positive control vector. After incubation overnight at 37°, bombarded tissues were stained for GUS activity. GUS expression was demonstrated by the blue spots on both the callus (Figure 6A) and leaf discs (Figure 6B) bombarded with pZSL11. As expected, the positive control 35S::GUS cassette was also expressed in both callus and leaf discs (Figure 6C, D).

### EXAMPLE 7

### Expression of Methionine Biosynthesis Genes by SAMS Promoter

The SAMS promoter was fused to two soybean cDNAs, one encoding ATP sulfurylase (ATPS) and a second encoding cystathionine-y-synthase (CGS1). The soybean ATPS and CGS 1 cDNAs were isolated from soybean embryo cDNA libraries using the same procedures as described in Example 1 and Example 2 for isolation of soybean SAMS cDNAs. The coding regions and the 3' untranslated region (UTR) of soybean ATPS and CGS1 genes were inserted into pZSL11 replacing the GUS gene. The resulting SAMS promoter::ATPS and SAMS promoter::CGS1 expression cassettes, SEQ ID NO:19 and SEQ ID NO:20, respectively, were inserted into binary vectors for *Arabidopsis* transformation and transformation was performed as described in Example 5. Transgenic *Arabidopsis* plants with soybean ATPS and CGS1 genes controlled by the SAMS promoter were analyzed by PCR for the presence of the transgenes and by RT-PCR for expression of the transgenes. Genomic DNA used for PCR analysis was prepared from *Arabidopsis* siliques and leaves using 7 M urea, 1.5 M NaCl, 50 mM Tris, pH 8.0, 20 mM EDTA, and 1 % N-lauroyl-sarcosine, followed by phenol extraction and ethanol precipitation. Primer sam-9 (SEQ ID NO:7) which is specific to SAMS promoter, and primers specific to the target genes, atps-1 (SEQ ID NO:8) for the ATPS gene and cgs-8 (SEQ ID NO:9) for the CGS 1 gene were used in PCR with Taq DNA polymerase (GIBCO BRL) to detect the existence of SAMS::ATPS and SAMS::CGS 1 in transgenic *Arabidopsis* plants.

| | |
|---|---|
| TTCGAGTATAGGTCACAATAGG | (SEQ ID NO:7) |
| CTTCGCTGAGGACATGGAC | (SEQ ID NO:8) |
| GAGTTGTCGCTGTTGTTCGAC | (SEQ ID NO:9) |

RNA samples used for RT-PCR were prepared with Trizol^{™} Reagent (GIBCO BRL). Antisense primers atps-4 (SEQ ID NO:10)and cgs-10 (SEQ ID NO:11) were used in reverse transcription reactions with SuperscriptII^{™} RT (GIBCO BRL) following the vendor's instruction.

| | |
|---|---|
| AACACAGCATCCGCATTGCG | (SEQ ID NO: 10) |
| AGGAGTGCAGAATCAGATCAG | (SEQ ID NO:11) |

The first strand cDNAs were used in PCR with primer pairs atps-3 (SEQ ID NO: 12) and atps-4 (SEQ ID NO: 10) for SAMS::ATPS transgenic plants, and cgs-9 (SEQ ID NO: 13) and cgs-10 for SAMS::CGS1 transgenic plants. PCR and RT-PCR products were resolved by agarose gel electrophoresis.

| | |
|---|---|
| GCTGATCGAACCAGATGGAG | (SEQ ID NO: 12) |
| CTGTACAGTTAAACAGTAGTTCT | (SEQ ID NO:13) |

All ten SAMS::CGS transgenic Arabidopsis harbored the SAM::CGS1 expression cassette as revealed by PCR with SAMS::CGS1-specific primers (Figure 7A). It was also revealed by the same analysis that all the ten SAMS::ATPS transgenic *Arabidopsis* plants contained the SAMS::ATPS expression cassette (Figure 7A). RT-PCR analysis detected CGS 1 transcripts and ATPS transcripts, respectively, in most of the transgenic plants (Figure 7B). This shows that the SAMS promoter is capable of driving expression of a variety of different genes in most or all cell types in transformed plants.

### EXAMPLE 8

### Induction of SAMS Promoter Activity by Methionine

Since SAMS catalyzes the reaction to synthesize S-adenosyl-L-methionine from methionine and ATP, free methionine levels might regulate SAMS promoter activity. To see if SAMS promoter is regulated by external methionine, the SAMS::GUS transgenic Arabidopsis seeds were germinated in the presence of either H₂O, 1 x Murashige and Skoog salt (GIBCO BRL), 0.01 mM methionine (Sigma), or 1 mM methionine. Ten days old seedlings from ten independent transgenic lines were analyzed for GUS activity according to the protocol described in Example 5. GUS activity for each treatment, in the order given above, for each transgenic line is shown in Figure 8. All lines responded similarly to the different treatments. Compared to the control of H₂O treamtment, SAMS activity was induced more than two-fold by 0.01 mM free methionine and inhibited about 40% on average by 1x MS salt. The induction effect of SAMS promoter by 1 mM methionine was less than that by 0.01 mM methionine, probably due to a toxic effect of the high methionine concentration; this toxic effect was indicated by the smaller sizes and shorter roots of the seedlings grown in the presence of 1 mM methionine. The toxic effect of high levels of methionine was even more apparent at 10 mM free methionine, since only a few Arabidopsis seeds were able to germinate and none survived in the presence of 10 mM free methionine.

## Claims

1. A chimeric gene comprising at least one heterologous nucleic acid fragment operably linked to an isolated nucleic acid fragment comprising a constitutive promoter which is capable of directing RNA production in many or all tissues of a plant, selected from the group consisting of:
(a) the nucleotide sequence set forth in SEQ ID NOs: 6, 14, 15, or 16; and
(b) a nucleotide sequence that is at least 80% identical to any one of the nucleotide sequences set forth in SEQ ID NOs: 6, 14, 15, or 16, based on the Clustal method of alignment;
wherein the heterologous nucleic acid fragment encodes a protein with herbicide-resistance or pathogen-resistance activity.

2. The chimeric gene of claim 1 wherein the heterologous nucleic acid fragment encodes a protein with herbicide-resistance activity.

3. A plant containing the chimeric gene of Claims 1 or 2.

4. The plant of Claim 3 wherein said plant is a monocot selected from the group consisting of corn, rice, wheat, barley and palm.

5. The plant of Claim 3 wherein said plant is a dicot selected from the group consisting of *Arabidopsis,* soybean, oilseed *Brassica,* peanut, sunflower, safflower, cotton, tobacco, tomato, potato, and cocoa.

6. The plant of claim 5 wherein said plant is soybean.

7. Seeds of the plants of Claims 3, 4, 5 or 6, wherein the seeds contain the chimeric gene of Claims 1 or 2.

8. A method of expressing at least one heterologous nucleic acid fragment in a plant cell which comprises:
(a) transforming a plant cell with the chimeric gene of Claims 1 or 2;
(b) growing fertile mature plants from the transformed plant cell of step (a);
(c) selecting plants of step (b) containing a transformed plant cell
wherein the heterologous nucleic acid fragment is expressed.

9. A method of selecting a plant cell having herbicide resistance which comprises:
(a) transforming a plant cell with the chimeric gene of Claim 2;
(b) selecting a transformed plant cell of step (a) wherein the heterologous nucleic acid fragment is expressed and wherein expression of the heterologous nucleic acid imparts herbicide-resistance to the plant cell.

10. A method of selecting a plant having herbicide resistance which comprises:
(a) transforming a plant cell with the chimeric gene of Claim 2;
(b) growing fertile mature plants from the transformed plant cell of step (a);
(c) selecting plants of step (b) containing a transformed plant cell
wherein the heterologous nucleic acid fragment is expressed and
wherein expression of the heterologous nucleic acid imparts herbicide-resistance to the plant.

11. A method of selecting a plant having herbicide resistance which comprises:
(a) transforming a plant cell with the chimeric gene of Claim 2;
(b) selecting a transformed plant cell of step (a) wherein the heterologous nucleic acid fragment is expressed and wherein expression of the heterologous nucleic acid imparts herbicide-resistance to the plant cell.
(c) growing fertile mature plants from the transformed plant cell of step (b);
(d) selecting plants of step (c) containing a transformed plant cell
wherein the heterologous nucleic acid fragment is expressed and
wherein expression of the heterologous nucleic acid imparts herbicide-resistance to the plant.

12. The method of any one of Claims 8 to 11 wherein the plant is a monocot selected from the group consisting of corn, rice, wheat, barley and palm.

13. The method of any one of Claims 8 to 11 wherein the plant is a dicot selected from the group consisting of *Arabidopsis,* soybean, oilseed *Brassica,* peanut, sunflower, safflower, cotton, tobacco, tomato, potato, and cocoa.

14. The method of Claim 13 wherein the plant is soybean.

15. An isolated nucleic acid fragment comprising a promoter wherein said promoter consists essentially of the nucleotide sequence set forth in SEQ ID NOs: 6, 14, 15, or 16 or said promoter consists essentially of a fragment or subfragment that is substantially similar and functionally equivalent to the nucleotide sequence set forth in SEQ ID NOs: 6, 14,15, or 16.

16. A chimeric gene comprising at least one heterologous nucleic acid fragment operably linked to the promoter of Claim 15 or Claim 24.

17. A plant containing the chimeric gene of Claim 16.

18. The plant of Claim 17 wherein said plant is a monocot selected from the group consisting of corn, rice, wheat, barley and palm.

19. The plant of Claim 17 wherein said plant is a dicot selected from the group consisting of *Arabidopsis,* soybean, oilseed *Brassica,* peanut, sunflower, safflower, cotton, tobacco, tomato, potato, and cocoa.

20. The plant of claim 19 wherein said plant is soybean.

21. Seeds of the plants of Claims 17, 18, 19 or 20.

22. A method of increasing or decreasing the expression of at least one heterologous nucleic acid fragment in a plant cell which comprises:
(a) transforming a plant cell with the chimeric gene of Claim 16;
(b) growing fertile mature plants from the transformed plant cell of step (a);
(c) selecting plants containing a transformed plant cell wherein the expression of the heterologous nucleic acid fragment is increased or decreased.

23. The method of Claim 22 wherein the plant is a monocot selected from the group consisting of corn, rice, wheat, barley and palm.

24. The method of Claim 23 wherein the plant is a dicot selected from the group consisting of *Arabidopsis,* soybean, oilseed *Brassica,* peanut, sunflower, safflower, cotton, tobacco, tomato, potato, and cocoa.

25. The method of Claim 24 wherein the plant is soybean.

26. An isolated nucleic acid fragment comprising a constitutive plant SAMS promoter.
